(19) Europäisches Patentamt
European Patent Office
Office européen des brevets

(11) EP 2 951 143 B1

(12) **EUROPÄISCHE PATENTSCHRIFT**

(45) Veröffentlichungstag und Bekanntmachung des Hinweises auf die Patenterteilung:
**30.11.2016 Patentblatt 2016/48**

(21) Anmeldenummer: **14701515.0**

(22) Anmeldetag: **22.01.2014**

(51) Int Cl.:
*C07C 45/50* (2006.01) *C07C 47/02* (2006.01)
*C07C 31/125* (2006.01) *C07C 29/156* (2006.01)
*C07C 31/135* (2006.01) *C07C 33/26* (2006.01)
*C07D 307/06* (2006.01)

(86) Internationale Anmeldenummer:
**PCT/EP2014/051208**

(87) Internationale Veröffentlichungsnummer:
**WO 2014/118046 (07.08.2014 Gazette 2014/32)**

(54) **VERFAHREN ZUR CARBONYLIERUNG VON OLEFINEN**
METHOD FOR THE CARBONYLATION OF OLEFINS
PROCÉDÉ DE CARBONYLATION D'OLÉFINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2013 DE 102013201669**

(43) Veröffentlichungstag der Anmeldung:
**09.12.2015 Patentblatt 2015/50**

(73) Patentinhaber: **Evonik Degussa GmbH**
**45128 Essen (DE)**

(72) Erfinder:
- **FRANKE, Robert**
**45772 Marl (DE)**
- **DYBALLA, Katrin Marie**
**45657 Recklinghausen (DE)**
- **HESS, Dieter**
**45770 Marl (DE)**
- **HAMERS, Bart**
**NL-5961 VG Horst (NL)**
- **FRIDAG, Dirk**
**45721 Haltern am See (DE)**
- **BELLER, Matthias**
**18211 Nienhagen (DE)**
- **FLEISCHER, Ivana**
**93057 Regensburg (DE)**
- **JACKSTELL, Ralf**
**27478 Cuxhaven Altenwalde (DE)**
- **PROFIR, Irina**
**18059 Rostock (DE)**
- **WU, Lipeng**
**City Xingtai**
**Hebei 055150 (CN)**

(56) Entgegenhaltungen:
**WO-A2-2007/078859 US-A- 4 602 116**

- **R. FRANKE; D. SELENT; A. BÖRNER: "Applied Hydroformylation", CHEM. REV., 2012, XP002722352, in der Anmeldung erwähnt**
- **TAKAHASHI, K.; YAMASHITA, M.; TANAKA, Y.; NOZAKI, K.: ANGEW. CHEM. INT. ED., Bd. 51, 2012, Seiten 4383-4387, XP002722353, in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 951 143 B1

## Beschreibung

Die Erfindung betrifft ein Verfahren zur Carbonylierung von Olefinen, bei dem wenigstens ein Olefin unter Zuführung von $H_2$ und CO in Gegenwart eines katalytischen Systems aus einem Ruthenium-Komplex und mindestens einem Liganden umgesetzt wird, wobei der Ligand ein organischer Phosphor-Liganden ist.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt (Schema 1). Als Katalysatoren in diesen Reaktionen werden häufig Verbindungen der Übergangsmetalle der VIII. Gruppe des Periodensystems der Elemente verwendet, insbesondere Rhodium- oder Cobaltkatalysatoren. Bekannte Liganden sind beispielsweise Verbindungen aus den Klassen der Phosphine, Phosphite und Phosphonite mit jeweils dreiwertigen Phosphor $P^{III}$. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI: 10.1021/cr3001803.

Aldehyde, insbesondere lineare Aldehyde wie Butyraldehyd, Valeraldehyd, Hexanal bzw. Octanal haben technische Bedeutung als Ausgangsprodukte für Weichmacheralkohole, Tenside, und Feinchemikalien.

Insgesamt wurden im Jahr 2008 mehr als 8 Mio Tonnen Oxo-Produkte mittels Hydroformylierung produziert.

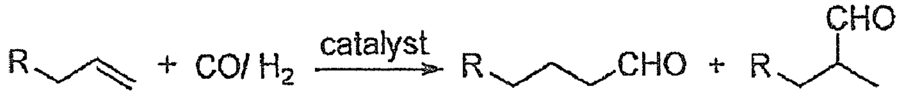


Schema 1

Katalysatoren, die im Rahmen der Hydroformylierungsreaktion allgemein verwendet werden, sind Rhodium- und Cobaltverbindungen in Gegenwart von Liganden. Leider stellen gerade Rhodium-Verbindungen vergleichsweise teure Edelmetall-Komplexe dar. So zählt Rhodium zu den teuersten Metallen überhaupt.

Besonders aktive Rhodiumkatalysatoren werden mit Phosphitliganden gebildet. Diese Liganden haben das Problem, daß sie empfindlich gegenüber einer Hydrolyse der P-O-Bindung sind und daß sie bei temperaturintensiver Aufarbeitung (z. B. Destillation) zur Zersetzung neigen. Stabilere Liganden sind Phosphane, die allerdings nur wenig aktive Hydroformylierungskatalysatoren bilden, um beispielsweise interne Olefine genügend schnell zu hydroformylieren. Ein generelles Problem bei der Entwicklung neuer Katalysatorsysteme für Hydroformylierungsreaktionen ist die Tatsache, dass keine Methoden existieren, rational die Aktivität und Selektivität von neuen Katalysatoren vorauszusagen.

Neue Katalysatoren sind sowohl für die Hydroformylierung von terminalen als auch internen Olefinen bzw. Olefingemischen interessant. Für Feinchemikalienanwendungen ist die Toleranz gegenüber funktionellen Gruppen ein wichtiges Kriterium. Daneben ist es wünschenswert, aktive Hydroformylierungskatalysatoren auf Basis kostengünstigerer Metalle zu entwickeln.

Eines dieser kostengünstigen Metalle ist das Ruthenium. Bis dato wurden in der Literatur jedoch nur vereinzelt Ruthenium-Komplexe als Hydroformylierungskatalysatoren beschrieben. Eine Übersicht der bis zum Jahre 1990 erschienen Resultate findet man in P. Kalck et al. in Adv. Organometal. Chem. 1991, 32, 121-146. Die dort beschriebenen Katalysatoren zeigen jedoch nur geringe - nicht wirtschaftliche - Aktivitäten und unbefriedigende Selektivitäten. Selbst neueste Arbeiten von Yamashita, Nozaki und Mitarbeitern (Angew. Chem. Int. Ed. 2012, 51, 4383-4387), die spezielle Ruthenium/Bisphosphin- bzw. Bisphosphit-Katalysatoren behandeln, sind mit Katalysatoraktivitäten (TOF) von <10 mol*mol $Ru^{-1}$*$h^{-1}$, von keiner wirtschaftlichen Relevanz.

Zusammenfassend kann festgestellt werden, daß kein Verfahren zur Carbonylierung mit Ruthenium-haltigen Katalysatoren bekannt ist, das hohe Katalysatoraktivitäten mit TOF >100 mol*mol $Ru^{-1}$*$h^{-1}$ aufweist und zugleich hohe Chemoselektivitäten (>90%) zum Aldehyd oder Alkohol erreicht. Beide Zielgrößen sind notwendig, um industrielle Umsetzungen zu erreichen.

Aus den oben genannten Gründen besteht ein großer Bedarf nach neuen verbesserten Katalysatorsystemen, die hohe Aktivität aufweisen und die nicht die Nachteile der bekannten Ruthenium-haltigen Katalysatoren zeigen.
Der Erfindung liegt die Aufgabe zugrunde, ein Verfahren zur Carbonylierung von Olefinen bereitzustellen, welches eine hohe Ausbeute erzielt, sowie eine hohe (Chemo-)Selektivität aufweist.

Die Aufgabe wird gelöst durch ein Verfahren nach Anspruch 1.

Verfahren zur Carbonylierung von Olefinen, dadurch gekennzeichnet, dass wenigstens ein Olefin unter Zuführung von $H_2$ und CO in Gegenwart eines katalytischen Systems aus einem Ruthenium-Komplex und mindestens einem Liganden umgesetzt wird, wobei der Ligand einen organischen Phosphor-Liganden einer der beiden allgemeinen Formeln (I) oder (II) darstellt:

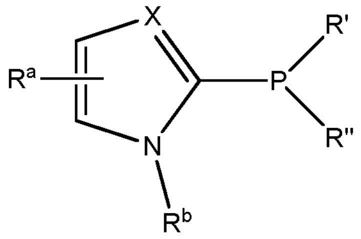

(I)

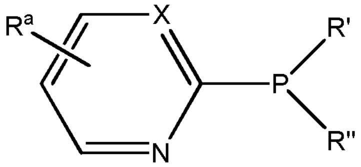

(II)

worin

R' und R" gleich oder verschieden sind und für einen Rest stehen, der ausgewählt ist aus der Gruppe bestehend aus:

($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, ($C_3$-$C_{12}$)-Cycloalkyl, ($C_3$-$C_{12}$)-Cycloalkyl-($C_1$-$C_{12}$)-Alkyl, ($C_3$-$C_{12}$)-Heterocycloalkyl, ($C_3$-$C_{12}$)-Heterocycloalkyl-($C_1$-$C_{12}$)-Alkyl, O-($C_1$-$C_{12}$)-Alkyl, O-($C_1$-$C_{12}$)-Heteroalkyl, O-($C_4$-$C_{14}$)-Aryl, O-($C_4$-$C_{14}$)-Aryl-($C_1$-$C_{14}$)-Alkyl, O-($C_3$-$C_{14}$)-Heteroaryl, O-($C_3$-$C_{14}$)-Heteroaryl-($C_1$-$C_{14}$)-Alkyl, O-($C_3$-$C_{12}$)-Cycloalkyl, O-($C_3$-$C_{12}$)-Cycloalkyl-($C_1$-$C_{12}$)-Alkyl, O-($C_3$-$C_{12}$)-Heterocycloalkyl, O-($C_3$-$C_{12}$)-Heterocycloalkyl-($C_1$-$C_{12}$)-Alkyl, wobei die genannten Alkyl-, Heteroalkyl-, Cycloalkyl-, Heterocycloalkyl-, Aryl- und Heteroarylgruppen optional ein- oder mehrfach substituiert sind und

$R^a$ und $R^b$ ausgewählt sind aus: Wasserstoff, Hydroxy, ($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, ($C_3$-$C_{12}$)-Cycloalkyl, ($C_3$-$C_{12}$)-Cycloalkyl-($C_1$-$C_{12}$)-Alkyl, ($C_3$-$C_{12}$)-Heterocycloalkyl, ($C_3$-$C_{12}$)-Heterocycloalkyl-($C_1$-$C_{12}$)-Alkyl, O-($C_1$-$C_{12}$)-Alkyl, O-($C_1$-$C_{12}$)-Heteroalkyl, O-($C_4$-$C_{14}$)-Aryl, O-($C_4$-$C_{14}$)-Aryl-($C_1$-$C_{14}$)-Alkyl, O-($C_3$-$C_{14}$)-Heteroaryl, O-($C_3$-$C_{14}$)-Heteroaryl-($C_1$-$C_{14}$)-Alkyl, O-($C_3$-$C_{12}$)-Cycloalkyl, O-($C_3$-$C_{12}$)-Cycloalkyl-($C_1$-$C_{12}$)-Alkyl, O-($C_3$-$C_{12}$)-Heterocycloalkyl, O-($C_3$-$C_{12}$)-Heterocycloalkyl-($C_1$-$C_{12}$)-Alkyl,

wobei die genannten Alkyl-, Heteroalkyl-, Cycloalkyl-, Heterocycloalkyl-, Aryl- und Heteroarylgruppen optional ein- oder mehrfach substituiert sind und
und wobei $R^a$ auch zur Bildung eines größeren kondensierten Ringes befähigt ist, und X ausgewählt ist aus: N, C.

Aryl steht für aromatische Kohlenwasserstoff-Reste, vorzugsweise mit 4 bis 10 C-Atomen, beispielsweise Phenyl- ($C_6H_5$-) oder Naphthyl- ($C_{10}H_7$-), wobei Phenyl besonders bevorzugt ist. Hierbei kann der Arylrest auch Teil einer größeren kondensierten Ringstruktur sein.

Alkyl steht für einen nicht verzweigten oder verzweigten aliphatischen Rest. Eine Alkylgruppe hat bevorzugt 1 bis 8 Kohlenstoffatome, besonders bevorzugt 1 bis 6 Kohlenstoffatome. Alkylgruppen sind z.B. Methyl, Ethyl, Propyl, Isopropyl, 1-Butyl, tert. Butyl, 1-Pentyl, 1-Hexyl.

Cycloalkyl steht für gesättigte cyclische Kohlenwasserstoffe, die ausschließlich KohlenstoffAtome im Ring enthalten.

Heteroalkyl steht für einen nicht verzweigten oder verzweigten aliphatischen Rest, der 1 bis 4, bevorzugt 1 oder 2 Heteroatome ausgewählt aus der Gruppe bestehend aus N, O, S und substituiertem N enthält.

Heteroaryl steht für einen Arylrest, in dem 1 bis 4, bevorzugt 1 oder 2 Kohlenstoffatome durch Heteroatome ausgewählt aus der Gruppe bestehend aus N, O, S und substituiertem N ersetzt sind. Hierbei kann der Heteroarylrest auch Teil einer größeren kondensierten Ringstruktur sein.
Heteroaryl steht bevorzugt für kondensierte Fünf- oder Sechsringe, wie beispielsweise Benzofuran, Isobenzofuran, Indol, Isoindol, Benzothiophen, Benzo(c)thiophen, Benzimidazol, Purin, Indazol, Imidazol, Benzoxazol, Chinolin, Isochinolin, Chinoxalin, Chinazolin, Cinnolin, Acridin.

Die genannten substituierten N können einfach substituiert sein, die Alkyl-, Heteroalkyl-, Cycloalkyl-, Heterocycloalkyl-, Aryl- und Heteroarylgruppen können ein oder mehrfach, besonders bevorzugt ein-, zwei- oder dreifach

substituiert sein durch Reste ausgewählt aus der Gruppe bestehend aus: Wasserstoff, $(C_1-C_{14})$-Alkyl, $(C_1-C_{14})$-Heteroalkyl, $(C_4-C_{14})$-Aryl, $(C_3-C_{14})$-Heteroaryl, $(C_3-C_{14})$-Heteroaryl-$(C_1-C_{14})$-Alkyl, $(C_3-C_{12})$-Cycloalkyl, $(C_3-C_{12})$-Heterocycloalkyl, Halogen (Fluor, Chlor, Brom, Iod), Hydroxy, $(C_1-C_{14})$-Alkoxy, $(C_4-C_{14})$-Aryloxy, $N((C_1-C_{14})\text{-Alkyl})_2$, $N((C_4-C_{14})\text{-Aryl})_2$, $N((C_1-C_{14})\text{-Alkyl})((C_4-C_{14})\text{-Aryl})$, wobei Alkyl, Aryl, Cycloalkyl, Heteroalkyl, Heteroaryl und Heterocycloalkyl die vorgenannten Bedeutungen haben.

Heterocycloalkyl für gesättigte cyclische Kohlenwasserstoffe, die 1 bis 4, bevorzugt 1 oder 2, Heteroatome ausgewählt aus der Gruppe bestehend aus N, O, S und substituiertem N enthalten.

In einer Variante des Verfahrens weist der Ligand die allgemeine Formel (I) auf.

In einer Variante des Verfahrens weist der Ligand die allgemeine Formel (II) auf.

In einer Variante des Verfahrens sind R' und R" ausgewählt aus: optional substituierten $(C_4-C_{14})$-Aryl-Rest, optional substituierten unverzweigten oder verzweigten aliphatischen Kohlenwasserstoffrest, optional substituierten Cycloalkylrest, optional substituierten Heteroarylrest und

$R^a$ und $R^b$ sind ausgewählt aus: Wasserstoff, $(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, wobei die genannten Alkyl-, Heteroalkyl-, Cycloalkyl-, Heterocycloalkyl-, Aryl- und Heteroarylgruppen optional ein- oder mehrfach substituiert sind

und wobei $R^a$ auch zur Bildung eines größeren kondensierten Ringes befähigt ist.

In einer anderen Variante des Verfahrens sind R' und R" ausgewählt aus: $(C_1-C_8)$-Alkyl, vorzugsweise Methyl, Ethyl, Propyl, Isopropyl und tert. Butyl, $(C_4-C_{14})$-Aryl-O-$(C_1-C_8)$-Alkyl, $(C_4-C_{14})$-Aryl, vorzugsweise Phenyl, Cyclohexyl, $(C_3-C_{14})$-Heteroaryl, $(C_3-C_{14})$-Heteroaryl-$(C_1-C_{12})$-Alkyl, vorzugsweise N-Methylimidazol und

$R^a$ und $R^b$ sind ausgewählt aus: Wasserstoff, $(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, wobei die genannten Alkyl-, Heteroalkyl-, Cycloalkyl-, Heterocycloalkyl-, Aryl- und Heteroarylgruppen optional ein- oder mehrfach substituiert sind und wobei $R^a$ auch zur Bildung eines größeren kondensierten Ringes befähigt ist.

In einer anderen Variante des Verfahrens sind R' und R" ausgewählt aus: $C_1$ bis $C_8$-Alkyl (insbesondere Methyl, Ethyl, Propyl, Isopropyl und tert. Butyl), Phenyl, Cyclohexyl, N-Methylimidazol.

In einer Variante des Verfahrens ist $R^b$ ausgewählt aus: Alkyl, Phenyl, $(C_4-C_{14})$-Aryl-O-$(C_1-C_{12})$-Alkyl und Alkylsubstituiertes Phenyl.

In einer Variante des Verfahrens ist $R^a$ gleich Wasserstoff.

In einer Variante des Verfahrens ist R' ausgewählt aus: Cyclohexyl, Phenyl, tert-Butyl und

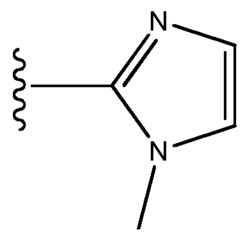

In einer Variante des Verfahrens ist R" ausgewählt aus: Cyclohexyl, Phenyl, tert-Butyl und

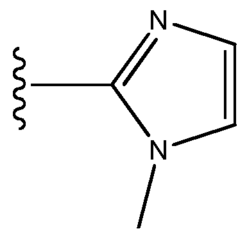

In einer Variante des Verfahrens ist der Ligand ausgewählt aus der Gruppe bestehend aus:

2-(Dicyclohexylphosphino)pyridin (L1a),
2-(Diphenylphosphino)pyridin (L1b),
2-(Dicyclohexylphosphino)-1-methyl-1H-imidazol (L2a),
2-(Di-terbutyl-phosphino)-1-methyl-1H-imidazol (L2b),

2-(Dicyclohexylphosphino)-1-(2-methoxyphenyl)-1H-imidazol (L2c),
2-(Dicyclohexylphosphino)-1-mesityl-1H-imidazol (L2d),
2-(Dicyclohexylphosphino)-1-methyl-1H-benzo[d]imidazol (L3).
2,2'-(Cyclohexylphosphinediyl)bis(1-methyl-1*H*-imidazol) (L4).

In einer Variante des Verfahrens wird der Ruthenium-Katalysator in situ ausgehend von einem Vorkomplex gebildet, wobei als Rutheniumquelle Ruthenium-enthaltende Salze und Komplexe als Vorstufe verwendet werden, die Rutheniumcarbonylhydrid-Komplexe bilden, vorzugsweise Ru(0)-carbonylverbindungen, Ru(II)- und Ru(III)-halogenide.

Als Rutheniumquelle können alle Ruthenium-enthaltende Salze und Komplexe als Vorstufe verwendet werden, die unter den Reaktionsbedingungen Rutheniumcarbonylhydrid-Komplexe bilden. Beispielhaft genannt werden Ru(0)-carbonylverbindungen, Ru(II)- und Ru(III)-halogenide. Die Rutheniumverbindungen können in unterschiedlichen Oxidationsstufen vorliegen, die mit Synthesegas und P-enthaltenden Liganden zu den entsprechenden aktiven Ruthenium(hydrido)(carbonyl)-Komplexen reagieren.

Eine besonders bevorzugte Vorstufe ist Trirutheniumdodecacarbonyl.

In einer Variante des Verfahrens wird dieses zur Umsetzung von Olefinen verwendet ausgewählt aus: Alkene, Cycloalkene, Carbonsäureester, aromatische Olefine mit jeweils einer Kohlenstoffanzahl von 2 bis 21.

Ungesättigte Verbindungen, die mit den genannten Katalysatorsystemen selektiv umgesetzt werden können, sind Alkine und Olefine, wobei Olefine bevorzugt sind. Besonders bevorzugt sind terminale Alkene, Cycloalkene und aromatische Olefine mit einer Kohlenstoffanzahl zwischen 2 und 21 und deren Gemische. Besonders bevorzugt sind Olefine mit 4 bis 12 Kohlenstoffatomen sowie deren Gemische.

Die verwendeten Olefine und Alkine können funktionalisiert sein. Ohne Anspruch auf Vollständigkeit seien hier als Substrate ungesättigte Alkohole, Ether, Amine, Ester, Carbonsäuren, Amide, Urethane, Halogenide, Aldehyde, Ketone und Epoxide genannt.

Das erfindungsgemäße Verfahren hat sich besonders für die Herstellung von Aldehyden oder Alkoholen mit 3 bis 21 Kohlenstoffatomen bewährt. Insbesondere ist die Herstellung von Aldehyden oder Alkoholen mit 5 bis 13 Kohlenstoffatomen bevorzugt. Überraschenderweise können bei dem erfindungsgemäßen Verfahren Aldehyde und Alkohole selektiv hergestellt werden. Höhere Reaktionstemperaturen oberhalb von 120 °C bevorzugen dabei eine selektive Bildung der Alkohole.

In einer Variante des Verfahrens wird bei einer Reaktionstemperatur im Bereich von 50 °C bis 200 °C gearbeitet, vorzugsweise in einem Bereich von 80 °C bis 180 °C, besonders bevorzugt von 100 °C bis 160 °C.

In einer Variante des Verfahrens wird bei einem Reaktionsdruck von 0,1 MPa bis 10,0 MPa gearbeitet, bevorzugt 0,5 MPa bis 10,0 MPa und besonders bevorzugt 1,0 MPa bis 8,0 MPa.

In einer Variante des Verfahrens liegt das Verhältnis Ruthenium : Ligand im Bereich von 1 : 1 bis 1 : 50, vorzugsweise im Bereich von 1 : 1 bis 1 : 10 besonders bevorzugt im Bereich von 1 : 1 bis 1 : 4.

In einer Variante des Verfahrens werden von 0,5 mol-% bis 0,001 mol-% Ruthenium bezogen auf das Olefin eingesetzt, vorzugsweise von 0,5 mol-% bis 0,05 mol-% und besonders bevorzugt werden von 0,2 mol-% bis 0,1 mol-% Ruthenium, bezogen auf das Olefinsubstrat, eingesetzt.

Für das erfindungsgemäße Verfahren können Lösungsmittel für den Katalysator eingesetzt werden. Als Lösungsmittel werden beispielsweise polare inerte organische Lösungsmittel oder/und Wasser verwendet. Zum Beispiel können dipolar aprotische Lösungsmittel, aliphatische Ether, Amide, aromatische Verbindungen, Alkohole und Ester, Ether sowie deren Gemische verwendet werden. Besonders bevorzugt sind Amide und Carbonate als Lösungsmittel wie beispielsweise N-Methylpyrrolidon (NMP) und Propylencarbonat (PC).

Additive die einen Selektivitätseinfluss ausüben können sind beispielsweise Erdalkali- und Alkalimetallhalogenide wie zum Beispiel LiCl, LiBr, $MgCl_2$ oder quartäre Stickstoffhalogenidsalze wie Tetra-n-butylammoniumchlorid oder Imidazoliumchlorid.

Bei dem erfindungsgemäßen Verfahren können Turnover-Werte [Turnover-Wert (TON) = Turnover-Frequenz (TOF) x Stunden Reaktionszeit] der Katalysatoren in der Größenordnung von >1.000 und mehr realisiert werden. Daher werden typischerweise von 0,5 mol-% bis 0,001 mol-% Ruthenium, bezogen auf das Olefinsubstrat, eingesetzt. Bevorzugt werden von 0,5 mol-% bis 0,05 mol-% und besonders bevorzugt von 0,2 mol-% bis 0,1 mol-% Ruthenium, bezogen auf das Olefinsubstrat, eingesetzt.

Aufgrund der signifikant verbesserten Katalysatoraktivitäten ist es bei dem erfindungsgemäßen Verfahren möglich, kleine Mengen an Katalysator zu verwenden, die den Prozess ökonomisch relevant machen.

Das erfindungsgemäße Verfahren ist insofern besonders überraschend und neu, da in der Vergangenheit keine hochselektiven Hydroformylierungen von Olefinen mit Ruthenium-Komplexen mit ausreichender Aktivität beschrieben wurden. Das beschriebene Verfahren zeigt hier erstmals, dass unter den erfindungsgemäßen Bedingungen gute Ausbeuten und Selektivitäten an Aldehyden oder Alkoholen möglich sind. Die besonderen Vorteile des neuen Verfahrens bestehen darin, dass Ruthenium als Katalysatormetall im Vergleich zu Rhodium deutlich kostengünstiger ist. Die erfindungsgemäß hergestellten Produkte können unter anderem eingesetzt werden als Zwischenprodukte für Weichmacheralkohole, als Tenside und als Vorprodukte für Pharmazeutika und Agrochemikalien sowie Bausteine für Polymere.

Beispiele:

Die nachstehenden Beispiele dienen zur Erläuterung des erfindungsgemäßen Verfahrens, ohne es darauf zu beschränken.

Allgemeine Arbeitsvorschrift zur Herstellung von Alkoholen aus Olefinen mittels eines Ruthenium/Phosphin-Katalysators:

Ein 25-mL Autoklav wird befüllt mit $Ru_3(CO)_{12}$ (25,6 mg; 40,0 μmol), Ligand (132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung nach Verdünnung mit Aceton mittels eines HP 6890 Gaschromatographs auf einer 30 m HP5 Säule analysiert. Hierfür wird folgende Methode verwendet: 10 Minuten bei 35 °C, dann heizen bis 280 °C mit 8 °C/min Aufheizrate, 6 Minuten bei 280 °C. Die Retentionszeiten einzelner Reaktanten und Produkten sind: 1-Okten (6,2 min), cis/trans 2-Oktene (6,9 min und 7,4 min), cis/trans 3- und 4-Oktene (6,0 min; 6,3 min und 6,5 min), Oktan (6,5 min), 1-Nonanal (19,5 min), isomere C9-Aldehyde (18,3 min; 18,5 min und 19,1 min), 1-Nonanol (21,1 min), isomere C9-Alkohole (19,8 min; 20,1 min und 20,2 min). Die Ausbeuten einzelner Produkte werden mit Hilfe der "Multiple Point Internal Standard GC Quantitation Method" bestimmt. Die Masse des Analyts wird mittels Gleichung (i) berechnet:

$$\text{Masse (Analyt)} = [\text{Masse (int. Standard)} \times \text{Signalfläche (int. Standard)} \times \text{Response-faktor}] / \text{Signalfläche (Analyt)} \qquad \text{(i)}$$

Der Responsefaktor wird über eine Kalibrierreihe mit 4 Analyt/Standard Lösungen mit verschiedenen bekannten Analyt- und Standardverhältnissen ermittelt. Für jede der Lösungen wird Responsefaktor als Verhältnis des Signals von Analyt (A) und Internem Standard (IS) auf bestimmte Konzentration normiert. Aus diesen Einzelwerten geht der Responsefaktor als deren Mittelwert hervor.

Die TON-(turnover number) und TOF-Werte (turnover frequency) werden mit den berechneten Ausbeuten mittels Gleichungen (ii) und (iii) bestimmt.

$$\text{TON} = (\text{Stoffmenge Produkt})/(\text{Stoffmenge Katalysator}) = \text{Ausbeute}^*(\text{Stoffmenge Substrat})/(\text{Stoffmenge Katalysator}) \qquad \text{(ii)}$$

$$\text{TOF} = \text{TON}/\text{Reaktionszeit} \qquad \text{(iii)}$$

wobei man unter Reaktionszeit die Zeit versteht, die zwischen dem Anfang des Gasverbrauchs und dem Anfang des Abkühlens liegt.

Allgemeine Arbeitsvorschrift zur Herstellung von Alkoholen aus Olefinen mittels eines Ruthenium/Phosphin-Katalysators:

Ein 25-mL Autoklav wird befüllt mit $Ru_3(CO)_{12}$ (25,6 mg; 40,0 μmol), Ligand (132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert.

Allgemeine Angaben siehe Anmerkung [a] der Tabelle 1.

In der nachfolgenden Tabelle 1 wird die Variation von Liganden der folgenden Strukturen dargestellt:

Liganden

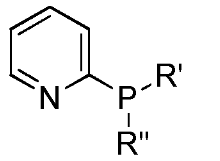

**1a** R' = R'' = Cy
**1b** R' = R'' = Ph

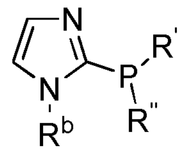

**2a** R' = R'' = Cy $R^b$ = Me
**2b** R' = R'' = $^t$Bu $R^b$ = Me
**2c** R' = R'' = Cy $R^b$ = 2-OMe($C_6H_4$)
**2d** R' = R'' = Cy $R^b$ = 2,4,6-$Me_3$($C_6H_2$)

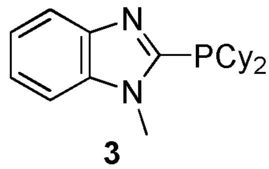

**3**

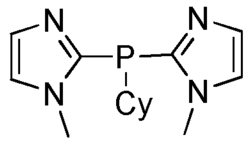

**4**

Das mögliche Produktspektrum des erfindungsgemäßen Verfahrens bei Verwendung von 1-Okten ergibt sich gemäß der Reaktion (1): 1-Nonanol (*n*), 2-Methyloctanol (*i*), 2-Ethylheptanol (i), 2-Propylhexanol (i), 1-Nonanal (*n*), 2-Methyloctanal (i), 2-Ethylheptanal (*i*), 2-Propylhexanal (*i*), Oktan, 2-Okten (*cis* und *trans*), 3-Okten (*cis* und *trans)* und 4-Okten (*cis* und *trans*).

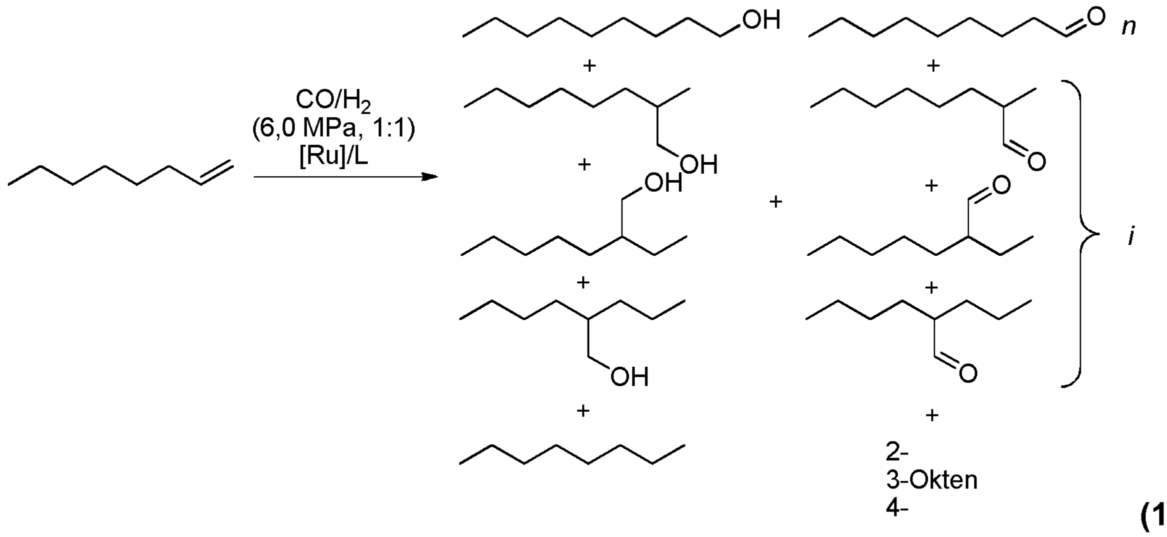


**(1)**

Tabelle 1: Ru-katalysierte Domino Hydroformylierung/Reduktion von 1-Okten

| Bsp.[a] | L | Alkohole (*n*/*i*) | Ausbeute [%][b] Aldehyde (*n*/*i*) | Alkan+Alkene[c] |
|---|---|---|---|---|
| 1 | **1a** | 52 (58:42) | 2[d] | 26 |
| 2 | **1b** | 15 (73:27) | 20 (60:40) | 44 |
| 3 | **2a** | 76 (86:14) | <0.5[d] | 7 (Alkan)[e] |
| 4 | **2b** | 32 (63:37) | 17 (35:65) | 10 |
| 5 | **2c** | 74 (84:16) | <0.5[d] | 5 (Alkan)[e] |
| 6 | **2d** | 75 (67:33) | 3[d] | 15 |
| 7 | **3** | 43 (91:9) | 25 (85:15) | 27 |
| 8 | **4** | 2[d] | 30 (93:7) | 27 |

(fortgesetzt)

| Bsp.[a] | L | Alkohole (*n*/*i*) | Ausbeute [%][b] Aldehyde (*n*/*i*) | Alkan+Alkene[c] |
|---|---|---|---|---|
| 9[f] | **2a** | 0 | 0 | 98 (1-Okten) |

[a] 20.0 mmol 1-Octen, 40.0 μmol $Ru_3(CO)_{12}$, 5.00 mmol LiCl, 132 μmol L, 0.5 mL $H_2O$, 4 mL NMP, 6,0 MPa $CO/H_2$ (1:1), 160 °C, 5 h.
[b] Bestimmt mit GC mit einem internen Standard (2.0 mL Isooktan).
[c] Kombinierte Ausbeute von Oktan und Oktenen.
[d] *n*/*i*-Verhältnis wurde nicht bestimmt.
[e] Nur Oktan wurde detektiert.
[f] Ohne $Ru_3(CO)_{12}$.

Tabelle 1 zeigt, dass die Ruthenium-katalysierte Hydroformylierung mit den Liganden der Formeln (L1a) bis (L4) in guten bis sehr guten Ausbeuten möglich ist. Dass diese Reaktion wirklich Ru-katalysiert verläuft, zeigt Beispiel 9 in der Tabelle 1. Hierbei wird die Reaktion mit Ligand, jedoch ohne Ruthenium durchgeführt und es konnte keine Hydroformylierung des Olefins beobachtet werden. Weiterhin zeigt Tabelle 1, dass die Reaktion mit guten n/iso-Selektivitäten durchgeführt werden kann.

*<u>Beispiel 1 (Tabelle 1):</u>*

Ein 25-mL Autoklav wird befüllt mit $Ru_3(CO)_{12}$ (25,6 mg; 40,0 μmol), 2-(Dicyclohexylphosphino)pyridin (36,3 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 52% mit einem *n*/*i*-Verhältnis von 58:42. Außerdem werden 2% an C9-Aldehyden und 26% an Oktenen und Oktan gefunden.

*<u>Beispiel 2 (Tabelle 1):</u>*

Ein 25-mL Autoklav wird befüllt mit $Ru_3(CO)_{12}$ (25,6 mg; 40,0 μmol), 2-(Diphenylphosphino)pyridin (34,7 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 15% mit einem *n*/*i*-Verhältnis von 73:27. Außerdem werden 20% an C9-Aldehyden mit einem *n*/*i*-Verhältnis von 60:40 und 44% an Oktenen und Oktan gefunden.

*<u>Beispiel 3 (Tabelle 1):</u>*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 76% mit einem *n*/*i*-Verhältnis von 86:14. Außerdem werden <0,5% an C9-Aldehyden und 7% an Oktan gefunden.

*<u>Beispiel 4 (Tabelle 1)</u>*:

Ein 25-mL Autoklav wird befüllt mit $Ru_3(CO)_{12}$ (25,6 mg; 40,0 μmol), 2-(Di-*tert*-butylphosphino)-1-methyl-1*H*-imidazol (29,9 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 32% mit einem *n*/*i*-Verhältnis von 63:37. Außerdem werden 17% an C9-Aldehyden mit einem *n*/*i*-Verhältnis von 35:65 und 10% an Oktenen

und Oktan gefunden.

*Beispiel 5 (Tabelle 1):*

Ein 25-mL Autoklav wird befüllt mit $Ru_3(CO)_{12}$ (25,6 mg; 40,0 μmol), 2-(Dicyclohexylphosphino)-1-(2-methoxyphenyl)-1 H-imidazol (48,9 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 74% mit einem *n*/*i*-Verhältnis von 84:16. Außerdem werden <0,5% an C9-Aldehyden und 5% an Oktan gefunden.

*Beispiel 6 (Tabelle* 1):

Ein 25-mL Autoklav wird befüllt mit $Ru_3(CO)_{12}$ (25,6 mg; 40,0 μmol), 2-(Dicyclohexylphosphino)-1-mesityl-1 H-imidazol (50,5 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 75% mit einem *n*/*i*-Verhältnis von 67:33. Außerdem werden 3% an C9-Aldehyden und 15% an Oktenen und Oktan gefunden.

*Beispiel 7 (Tabelle 1):*

Ein 25-mL Autoklav wird befüllt mit $Ru_3(CO)_{12}$ (25,6 mg; 40,0 μmol), 2-(Dicyclohexylphosphino)-1-methyl-1 H-benzo[d]imidazol (43,3 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 43% mit einem *n*/*i*-Verhältnis von 91:9. Außerdem werden 25% an C9-Aldehyden mit einem *n*/*i*-Verhältnis von 85:15 und 27% an Oktenen und Oktan gefunden.

*Beispiel 8 (Tabelle* 1):

Ein 25-mL Autoklav wird befüllt mit $Ru_3(CO)_{12}$ (25,6 mg; 40,0 μmol), 2,2'-(Cyclohexylphosphinediyl)bis(1-methyl-1 H-imidazol) (36,5 mg; 132 μmol) und LiCl (212 mg; 5,00 mmol). NMP (4 mL), $H_2O$ (0,5 mL; 0,5 g; 28 mmol) und 1-Octen (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 2%. Außerdem werden 30% an C9-Aldehyden mit einem *n*/*i*-Verhältnis von 93:7 und 27% an Oktenen und Oktan gefunden.

*Beispiel 9 (Tabelle* 1):

Ein 25-mL Autoklav wird befüllt mit 2-(Dicyclohexylphosphino)-1-methyl-1 H-imidazol (36,7 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Es werden 98% an 1-Okten gefunden.

In folgenden Beispielen der Tabelle zwei wird Einfluss verschiedener Parameter, darunter Temperatur, Menge des zugegebenen Wassers und LiCl, untersucht. In Beispielen der Tabelle drei wird Einwirkung des Lösungsmittels (LM), der Temperatur und des Druckes auf die selektive Umsetzung von Olefinen zu Aldehyden gezeigt.

Tabelle 2: Ru-katalysierte Domino Hydroformylierung/Reduktion von 1-Okten

| Bsp.[a] | T [°C] | Zeit [h] | $H_2O$ [mol%] | Ausbeute [%][b] Alkohole (*n*/*i*) | Aldehyde (*n*/*i*) | Alkan+Alkene[c] |
|---|---|---|---|---|---|---|
| 10 | 160 | 5 | 140 | 76 (86:14) | <0.5[d] | 7 (Alkan)[e] |
| 11 | 160 | 5 | 0 | 44 (77:23) | 4 (50:50) | 14 |
| 12 | 160 | 5 | 280 | 87 (90:10) | 2[d] | 8 (Alkan)[e] |
| 13 | 160 | 5 | 560 | 74 (92:8) | 3 (67:33) | 14 |
| 14 | 130 | 20 | 280 | 90 (88:12) | 1[d] | 3 (Alkan)[e] |
| 15 | 120 | 22 | 280 | 78 (90:10) | 5 (80:20) | 3 (Alkan)[e] |
| 16 | 100 | 20 | 140 | 18 (94:6) | 66 (88:12) | 9 |
| 17 | 80 | 24 | 140 | 0 | 75 (93:7) | 20 |
| 18[f] | 160 | 5 | 0 | 77 (88:12) | <0.5[d] | 5 (Alkan)[e] |
| 19[f] | 160 | 5 | 140 | 88 (86:14) | <0.5[d] | 5 (Alkan)[e] |
| 20[g] | 130 | 20 | 280 | 68 (90:10) | 8 (75:25) | 6 |
| 21[h] | 130 | 20 | 280 | 37 (95:5) | 54 (85:15) | 7 |
| 22[h] | 100 | 20 | 280 | 6 (95:5) | 83 (89:11) | 9 |

[a] Soweit nicht anders beschrieben: 20,0 mmol 1-Okten, 40,0 μmol $Ru_3(CO)_{12}$, 5,00 mmol LiCl, 132 μmol 2-(Dicyclohexylphosphino)-1-methyl-1H-imidazol, $H_2O$, 4 mL NMP, 6,0 MPa $CO/H_2$ (1:1) bei angegebener Temperatur und Reaktionszeit.
[b] Bestimmt mit GC mit einem internen Standard (2,0 mL Isooktan).
[c] Kombinierte Ausbeute von Oktan und Oktenen.
[d] *n*/*i*-Verhältnis wurde nicht bestimmt.
[e] Nur Oktan wurde detektiert.
[f] Mit $CO/H_2$ (60 bar, 1:2).
[g] Mit 0.1 mol% $Ru_3(CO)_{12}$ (20,0 μmol), 2,50 mmol LiCl, 76 μmol 2-(Dicyclohexylphosphino)-1-methyl-1H-imidazol.
[h] Ohne LiCl.

*Beispiel 10 (Tabelle 2):* entspricht Beispiel 3.

*Beispiel 11 (tabelle 2):*

Ein 25-mL Autoklav wird befüllt mit $Ru_3(CO)_{12}$ (25,6 mg; 40,0 μmol), 2-(Dicyclohexylphosphino)-1-methyl-1H-imidazol (36,7 mg; 132 μmol) und LiCl (212 mg; 5,00 mmol). NMP (4 mL), und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 44% mit einem *n*/*i*-Verhältnis von 77:23. Außerdem werden 4% an C9-Aldehyden mit einem *n*/*i*-Verhältnis von 50:50 und 14% an Oktenen und Oktan gefunden.

*Beispiel 12 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1H-imidazol (36,7 mg; 132 μmol) und LiCl (212 mg; 5,00 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 87% mit einem *n*/*i*-Verhältnis von 90:10. Außerdem werden 2% an C9-Aldehyden und 8% an Oktan gefunden.

*Beispiel 13 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (2,0 mL; 2,0 g; 112 mmol) und 1-Okten

(3,1 mL; 2,2 g; 20 mmol) werden hinzugegeben und es wird Synthesegas ($CO/H_2$ 1:1 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 74% mit einem *n*/*i*-Verhältnis von 92:8. Außerdem werden 3% an C9-Aldehyden mit einem *n*/*i*-Verhältnis von 67:33 und 14% an Oktenen und Oktan gefunden.

*Beispiel 14 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 90% mit einem *n*/*i*-Verhältnis von 88:12. Außerdem werden 1% an C9-Aldehyden und 3% an Oktan gefunden.

*Beispiel 15 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 120 °C erwärmt und 22 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 78% mit einem *n*/*i*-Verhältnis von 90:10. Außerdem werden 5% an C9-Aldehyden mit einem *n*/*i*-Verhältnis von 80:20 und 3% an Oktan gefunden.

*Beispiel 16 (tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 100 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 18% mit einem *n*/*i*-Verhältnis von 94:6. Außerdem werden 66% an C9-Aldehyden mit einem *n*/*i*-Verhältnis von 88:12 und 9% an Oktenen und Oktan gefunden.

*Beispiel 17 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 80 °C erwärmt und 24 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 0%. Es werden 75% an C9-Aldehyden mit einem *n*/*i*-Verhältnis von 93:7 und 20% an Oktenen und Oktan gefunden.

*Beispiel 18 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (4 mL), und 1-Okten (3,1 mL; 2,2 g; 20 mmol) werden hinzugegeben und es wird Synthesegas ($CO/H_2$ 1:2 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 77% mit einem *n*/*i*-Verhältnis von 88:12. Außerdem werden <0,5% an C9-Aldehyden und 5% an Oktan gefunden.

*Beispiel 19 (Tabelle 2):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (4 mL), $H_2O$ (0,5 mL; 0,5 g; 28 mmol) und 1-Okten (3,1 mL; 2,2 g; 20 mmol) werden hinzugegeben und es wird Synthesegas ($CO/H_2$ 1:2 6,0 MPa) aufgepresst. Die Reaktionsmischung wird auf 160 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 88% mit einem *n*/*i*-Verhältnis von 86:14. Außerdem werden <0,5% an C9-Aldehyden und 5% an Oktan gefunden.

*Beispiel 20 (Tabelle 2):*

Ein 25-mL Autoklav wird befüllt mit $Ru_3(CO)_{12}$ (12,8 mg; 20,0 μmol), 2-(Dicyclohexylphosphino)-1-methyl-1H-imidazol (18,3 mg; 76 μmol) und LiCl (106 mg; 2,50 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 68% mit einem *n*/*i*-Verhältnis von 90:10. Außerdem werden 8% an C9-Aldehyden mit einem *n*/*i*-Verhältnis von 75:25 und 6% an Oktenen und Oktan gefunden.

*Beispiel 21 (Tabelle 2):*

Ein 25-mL Autoklav wird befüllt mit $Ru_3(CO)_{12}$ (25,6 mg; 40,0 μmol) und 2-(Dicyclohexylphosphino)-1-methyl-1 H-imidazol (36,7 mg; 132 μmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 37% mit einem *n*/*i*-Verhältnis von 95:5. Außerdem werden 54% an C9-Aldehyden mit einem *n*/*i*-Verhältnis von 85:15 und 7% an Oktenen und Oktan gefunden.

*Beispiel 22 (Tabelle 2):*

Ein 25-mL Autoklav wird befüllt mit $Ru_3(CO)_{12}$ (25,6 mg; 40,0 μmol) und 2-(Dicyclohexylphosphino)-1-methyl-1 H-imidazol (36,7 mg; 132 μmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 100 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 6% mit einem *n*/*i*-Verhältnis von 95:5. Außerdem werden 83% an C9-Aldehyden mit einem *n*/*i*-Verhältnis von 89:11 und 9% an Oktenen und Oktan gefunden.

Tabelle 3: Ru-katalysierte Hydroformylierung von 1-Okten

| Bsp.[a] | T [°C] | Zeit [h] | LM | $H_2/CO$ [bar] | Ausbeute [%][b] | | |
|---|---|---|---|---|---|---|---|
| | | | | | Alkohole (*n*/*i*) | Aldehyde (*n*/*i*) | Alkan+Alkene[c] |
| 23 | 100 | 3 | NMP/ $H_2O$ | 30/30 | 0 | 23 (92:8) | 76 |
| 24 | 100 | 3 | PC/$H_2O$ | 30/30 | 0 | 10 (94:6) | 89 |
| 25 | 100 | 3 | PC | 30/30 | 0 | 32 (94:6) | 69 |
| 26 | 100 | 3 | PC | 40/20 | 0 | 66 (96:4) | 32 |
| 27[e] | 100 | 8 | PC | 40/20 | 0 | 74 (95:5) | 23 |
| 28[e] | 130 | 1 | PC | 40/20 | 0 | 61 (95:5) | 35 |
| 29[f] | 130 | 0,6 | PC | 40/20 | 0 | 65 (95:5) | 33 |

(fortgesetzt)

| Bsp.[a] | T [°C] | Zeit [h] | LM | $H_2$/CO [bar] | Ausbeute [%][b] Alkohole (*n*/*i*) | Aldehyde (*n*/*i*) | Alkan+Alkene[c] |
|---|---|---|---|---|---|---|---|
| 30[g] | 130 | 40 | PC | 40/20 | 14 (82:18) | 63 (73:27) | 17 |

[a] Soweit nicht anders beschrieben: 50,0 mmol 1-Okten, 16,7 μmol $Ru_3(CO)_{12}$, 55,1 μmol 2-(Dicyclohexylphosphino)-1-methyl-1 H-imidazol, $H_2O$, 25 mL Lösungsmittel (LM), angegebener Druck CO/$H_2$ bei angegebener Temperatur und Reaktionszeit in einem 100 mL Autoklav.
[b] Bestimmt mit GC mit einem internen Standard (2,0 mL Isooktan).
[c] Kombinierte Aubeute von Oktan und Oktenen.
[d] *n*/*i*-Verhältnis wurde nicht bestimmt.
[e] Mit 8,3 μmol $Ru_3(CO)_{12}$, 28 μmol 2-(Dicyclohexylphosphino)-1-methyl-1H-imidazol.
[f] Mit 8,3 μmol $Ru_3(CO)_{12}$, 28 μmol 2-(Dicyclohexylphosphino)-1-(2-methoxyphenyl)-1 H-imidazol.
[g] Mit 50,0 mmol 2-Okten.

*Beispiel 23 (Tabelle 3):*

Ein 100-mL Autoklav wird befüllt mit $Ru_3(CO)_{12}$ (10,7 mg; 16,7 μmol) und 2-(Dicyclohexylphosphino)-1-methyl-1H-imidazol (15,3 mg; 55,1 μmol). NMP (25 mL), $H_2O$ (2,5 mL; 2,5 g; 140 mmol) und 1-Okten (7,85 mL; 5,61 g; 50,0 mmol) werden hinzugegeben und es wird Synthesegas (CO/$H_2$ 1:1 6,0 MPa) aufgepresst. Die Reaktionsmischung wird auf 100 °C erwärmt und 3 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 23% mit einem *n*/*i*-Verhältnis von 92:8 Außerdem werden 71% an 1-Octen und 5% an 2-Octenen gefunden.

*Beispiel 24 (Tabelle 3):*

Ein 100-mL Autoklav wird befüllt mit $Ru_3(CO)_{12}$ (10,7 mg; 16,7 μmol) und 2-(Dicyclohexylphosphino)-1-methyl-1H-imidazol (15,3 mg; 55,1 μmol). PC (25 mL), $H_2O$ (2,5 mL; 2,5 g; 140 mmol) und 1-Okten (7,85 mL; 5,61 g; 50,0 mmol) werden hinzugegeben und es wird Synthesegas (CO/$H_2$ 1:1 6,0 MPa) aufgepresst. Die Reaktionsmischung wird auf 100 °C erwärmt und 3 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 10% mit einem *n*/*i*-Verhältnis von 94:6. Außerdem werden 84% an 1-Octen und 5% an 2-Octenen gefunden.

*Beispiel 25 (Tabelle 3):*

Ein 100-mL Autoklav wird befüllt mit $Ru_3(CO)_{12}$ (10,7 mg; 16,7 μmol) und 2-(Dicyclohexylphosphino)-1-methyl-1H-imidazol (15,3 mg; 55,1 μmol). PC (25 mL), und 1-Okten (7,85 mL; 5,61 g; 50,0 mmol) werden hinzugegeben und es wird Synthesegas (CO/$H_2$ 1:1 6,0 MPa) aufgepresst. Die Reaktionsmischung wird auf 100 °C erwärmt und 3 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 32% mit einem *n*/*i*-Verhältnis von 94:6. Außerdem werden 60% an 1-Octen, 8% an 2-Octenen und 1% an 3-Octenen gefunden.

*Beispiel 26 (Tabelle 3):*

Ein 100-mL Autoklav wird befüllt mit $Ru_3(CO)_{12}$ (10,7 mg; 16,7 μmol) und 2-(Dicyclohexylphosphino)-1-methyl-1H-imidazol (15,3 mg; 55,1 μmol). PC (25 mL), und 1-Okten (7,85 mL; 5,61 g; 50,0 mmol) werden hinzugegeben und es wird Synthesegas (CO/$H_2$ 1:2 6,0 MPa) aufgepresst. Die Reaktionsmischung wird auf 100 °C erwärmt und 3 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 66% mit einem *n*/*i*-Verhältnis von 96:4. Außerdem werden 1% an 1-Octen, 28% an 2-Octenen und 3% an 3-Octenen gefunden.

*Beispiel 27 (Tabelle 3):*

Ein 100-mL Autoklav wird befüllt mit $Ru_3(CO)_{12}$ (5,3 mg; 8,3 μmol) und 2-(Dicyclohexylphosphino)-1-methyl-1 H-imidazol (7,7 mg; 28 μmol). PC (25 mL), und 1-Okten (7,85 mL; 5,61 g; 50,0 mmol) werden hinzugegeben und es wird Synthesegas ($CO/H_2$ 1:2 6,0 MPa) aufgepresst. Die Reaktionsmischung wird auf 100 °C erwärmt und 8 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 74% mit einem *n/i*-Verhältnis von 95:5. Außerdem werden 23% an Oktenen und Oktan gefunden.

*Beispiel 28 (Tabelle 3):*

Ein 100-mL Autoklav wird befüllt mit $Ru_3(CO)_{12}$ (5,3 mg; 8,3 μmol) und 2-(Dicyclohexylphosphino)-1-methyl-1 H-imidazol (7,7 mg; 28 μmol). PC (25 mL), und 1-Okten (7,85 mL; 5,61 g; 50,0 mmol) werden hinzugegeben und es wird Synthesegas ($CO/H_2$ 1:2 6,0 MPa) aufgepresst. Die Reaktionsmischung wird auf 130 °C erwärmt und 1 Stunde bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 61% mit einem *n/i*-Verhältnis von 95:5 (entspricht TON = 1220; TOF = 1220 mol*mol $Ru^{-1}*h^{-1}$). Außerdem werden 35% an Oktenen und Oktan gefunden.

*Beispiel 29 (Tabelle 3):*

Ein 100-mL Autoklav wird befüllt mit $Ru_3(CO)_{12}$ (5,3 mg; 8,3 μmol) und 2-(Dicyclohexylphosphino)-1-(2-methoxyphenyl)-1 H-imidazol (10,4 mg; 28 μmol). PC (25 mL), und 1-Okten (7,85 mL; 5,61 g; 50,0 mmol) werden hinzugegeben und es wird Synthesegas ($CO/H_2$ 1:2 6,0 MPa) aufgepresst. Die Reaktionsmischung wird auf 130 °C erwärmt und 35 Minuten bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 65% mit einem *n/i*-Verhältnis von 95:5 (entspricht TON = 1300; TOF = 2167 mol*mol $Ru^{-1}*h^{-1}$). Außerdem werden 33% an Oktenen und Oktan gefunden.

*Beispiel 30 (Tabelle 3):*

Ein 100-mL Autoklav wird befüllt mit $Ru_3(CO)_{12}$ (10,7 mg; 16,7 μmol) und 2-(Dicyclohexylphosphino)-1-methyl-1H-imidazol (15,3 mg; 55,1 μmol). PC (25 mL), und 2-Okten (7,85 mL; 5,61 g; 50,0 mmol) werden hinzugegeben und es wird Synthesegas ($CO/H_2$ 1:2 6,0 MPa) aufgepresst. Die Reaktionsmischung wird auf 130 °C erwärmt und 40 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Aldehyden beträgt 63% mit einem *n/i*-Verhältnis von 73:27. Außerdem werden 14% an C9-Alkoholen mit einem *n/i*-Verhältnis von 82:18 und 17% an Oktenen und Oktan gefunden.

In den nachfolgenden Beispielen der Tabelle 4 sind die ungesättigten Ausgangsverbindungen, die erhaltenen Alkohole und die weiteren entsprechenden Parameter aufgeführt.

Die Spalte n/iso zeigt die Anteile Produkt mit endständiger Hydroxymethyl (n) und nichtendständiger Hydroxymethyl (iso) an.

In der nachfolgenden Gleichung (2) ist der allgemeine Reaktionsverlauf dargestellt. Die Substituenten $R_1$, $R_2$ und $R_3$ entsprechen den Gruppen oder Teilen der Verbindungen, die in den Beispielen 31 bis 43 aus den Spalten "Substrat" und "Produkt" zu entnehmen sind.

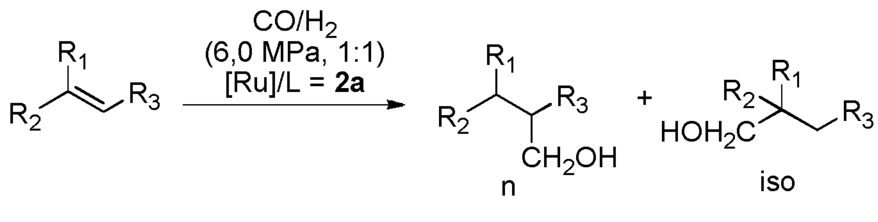

n
iso

**(2)**

Folgende allgemeine Reaktionsführung ([a]) wurde für die Beispiele 31-43 der Tabelle 4 angewendet, soweit nicht anders beschrieben: Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56

mmol) und Substrat (20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert.

Tabelle 4: Ru-katalysierte Domino Hydroformylierung/Reduktion von Alkenen

| Bsp.[a] | Substrat | Hauptprodukt | Aubeute [%][b] | | | |
|---|---|---|---|---|---|---|
| | | | Alkohole[c] (*n*/*i*) | Aldehyde (*n*/*i*) | Alkene[d] | Alkan |
| 31 | 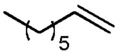 | 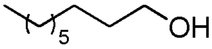 | 90 [81] (88:12) | 1[e] | 0 | 3 |
| 32 | 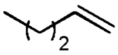 | 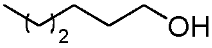 | 82 [75] (89:11) | 2[e] | -[f] | -[f] |
| 33 | 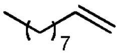 | 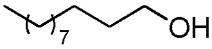 | 85 [83] (89:11) | 4 (75:25) | 2 | 6 |
| 34 | 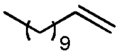 | 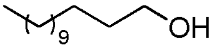 | 88 [81] (89:11) | 3 (67:33) | 3 | 6 |
| 35[g] | 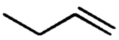 | 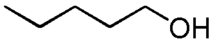 | 83 (85:15) | 15 (73:27) | -[f] | -[f] |
| 36 | 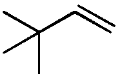 | 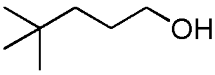 | >99 [87] (>99:1) | 0 | -[f] | -[f] |
| 37 | 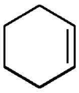 | 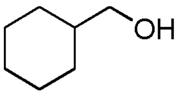 7 | 79 [76] | 3 | 1 | 0 |
| 38 | 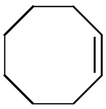 | 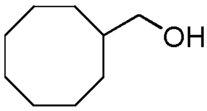 | 28 | 8 | 41 | 2 |
| 39 | 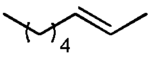 | 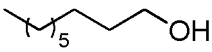 | 59 (66:34) | 14 (57:43) | 10 | 5 |
| 40 | 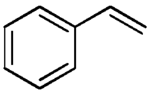 | 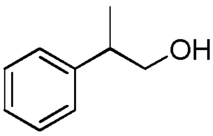 | 83 [80] (40:60) | 0 | 0 | 10 |
| 41 | 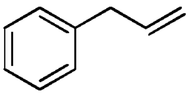 | 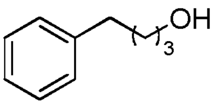 | 89 (64:36) | 0 | 0 | 9 |
| 42[h] | 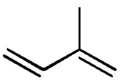 | 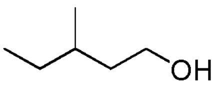 | >99 (96:4) | 0 | -[f] | -[f] |

(fortgesetzt)

| Bsp.[a] | Substrat | Hauptprodukt | Aubeute [%][b] Alkohole[c] (n/i) | Aldehyde (n/i) | Alkene[d] | Alkan |
|---|---|---|---|---|---|---|
| 43 | 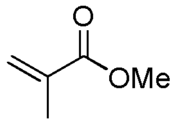 | 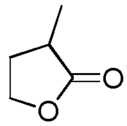 | 74 | 0 | 0 | -[f] |

[a] Soweit nicht anders beschrieben: 20,0 mmol Substrat, 40,0 μmol $Ru_3(CO)_{12}$, 132 μmol 2-(Dicyclohexylphosphino)-1-methyl-1 H-imidazol, 5,00 mmol LiCl, 1 mL $H_2O$, 3 mL NMP, 6,0 MPa $CO/H_2$ (1:1), 130 °C, 20 h in einem 25 mL Autoklav.
[b] Bestimmt mit GC mit einem internen Standard (2,0 mL Isooktan).
[c] Isolierte Ausbeute an Alkoholen ist in eckigen Klammern angegeben.
[d] Alle Isomere.
[e] *n*/*i*-Verhältnis wurde nicht bestimmt.
[f] Verbindung nicht detektierbar mit GC.
[g] Durchgeführt mit 103 mmol 1-Buten, 200 μmol $Ru_3(CO)_{12}$, 25.0 mmol LiCl, 660 μmol 2-(Dicyclohexylphosphino)-1-methyl-1H-imidazol, 275 mmol $H_2O$, 15 mL NMP, mit konstantem Druck 6,0 MPa $CO/H_2$ (1:1), 130 °C, 20 h in einem 100 mL Autoklav.
[h] Zusammensetzung der Produkte: 3-Methyl-1-pentanol, 4-Methyl-1-pentanol, 2,3-Dimethyl-1-butanol (82:14:4).

*Beispiel 31 (Tabelle 4):*

Entspricht Beispiel 14. 1-Nonanol wurde nach der Extraktion des Lösungsmittels mit Wasser und anschließender Kugelrohr-Destillation (Siedepunkt 100-105 °C bei 1,3 kPa) in 81% Ausbeute und 95% Reinheit erhalten.

*Beispiel 32 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und 1-Penten (2,2 mL; 1,4 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C6-Alkoholen beträgt 82% mit einem *n*/*i*-Verhältnis von 89:11. Außerdem werden 2% an C6-Aldehyden gefunden. 1-Hexanol wurde nach der Extraktion des Lösungsmittels mit Wasser und anschließender Kugelrohr-Destillation (Siedepunkt 70-75 °C bei 3,0 kPa) in 75% Ausbeute und 95% Reinheit erhalten.

*Beispiel* 33 *(Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (2,5 mL), $H_2O$ (1,0 mL; 1,0 g; 56 mmol) und 1-Decen (3,8 mL; 2,8 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C11-Alkoholen beträgt 85% mit einem *n*/*i*-Verhältnis von 89:11. Außerdem werden 4% an C11-Aldehyden mit einem *n*/*i*-Verhältnis von 75:25, 2% isomere Decene und 6% n-Dekan gefunden. 1-Undekanol wurde nach der Extraktion des Lösungsmittels mit Wasser und anschließender Kugelrohr-Destillation (Siedepunkt 135-140 °C bei 1,5 kPa) in 83% Ausbeute und 97% Reinheit erhalten.

*Beispiel 34 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (2 mL), $H_2O$ (1,0 mL; 1,0 g; 56 mmol) und 1-Dodecen (4,4 mL; 3,4 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav

abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C13-Alkoholen beträgt 88% mit einem *n*/*i*-Verhältnis von 89:11. Außerdem werden 3% an C11-Aldehyden mit einem *n*/*i*-Verhältnis von 67:33, 3% isomere Decene und 6% n-Dekan gefunden. 1-Tridekanol wurde nach der Extraktion des Lösungsmittels mit Wasser und anschließender Kugelrohr-Destillation (Siedepunkt 150-155 °C bei 1,5 kPa) in 81% Ausbeute und 93% Reinheit erhalten.

*Beispiel 35 (Tabelle 4):*

Ein 100-mL Autoklav wird befüllt mit $Ru_3(CO)_{12}$ (128 mg; 200 μmol), 2-(Dicyclohexylphosphino)-1-methyl-1 H-imidazol (183 mg; 660 μmol) und LiCl (1,06 g; 25,0 mmol). NMP (15 mL), $H_2O$ (5,0 mL; 5,0 g; 275 mmol) und 1-Buten (5,8 g; 102 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur und konstantem Druck gerührt. Danach wird der Autoklav abgekühlt und Gas abgelassen. Nach der Zugabe des internen Standards (Isooktan, 10,0 mL; 6,92 g; 60,6 mmol) wird die Reaktionsmischung gaschromatographisch analysiert. Die Ausbeute an C5-Alkoholen beträgt 83% mit einem *n*/*i*-Verhältnis von 85:15. Außerdem werden 15% an C11-Aldehyden mit einem *n*/*i*-Verhältnis von 73:27 gefunden.

*Beispiel* 36 *(Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und 3,3-Dimethyl-1-buten (2,6 mL; 2,3 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C6-Alkoholen beträgt >99% mit einem *n*/*i*-Verhältnis von >99:1. 3,3-Dimethylpentan-1-ol wurde nach der Extraktion des Lösungsmittels mit Wasser und anschließender Kugelrohr-Destillation (Siedepunkt 70-75 °C bei 2,0 kPa) in 87% Ausbeute und 98% Reinheit erhalten.

*Beispiel 37 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Cyclohexen (2,0 mL; 1,6 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an Cyclohexylmethanol beträgt 79%. Außerdem werden 3% an Cyclohexankarbaldehyd und 1% Cyclohexen gefunden. Cyclohexylmethanol wurde nach der Extraktion des Lösungsmittels mit Wasser und anschließender Kugelrohr-Destillation (Siedepunkt 85-90 °C bei 2,0 kPa) in 76% Ausbeute erhalten.

*Beispiel* 38 *(Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Cycloocten (2,6 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an Cyclooktylmethanol beträgt 28%. Außerdem werden 8% an Cyclooctankarbaldehyd, 41% Cycloocten und 2% Cyclooktan gefunden.

*Beispiel* 39 *(Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und 2-Octen (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 59% mit einem *n*/*i*-Verhältnis von 66:34. Außerdem werden 14% an C9-Aldehyden mit einem *n*/*i*-Verhältnis von 57:43, 10% Octene und 5% Oktan gefunden.

*Beispiel 40 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Styrol (2,3 mL; 2,1 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an entsprechenden Alkoholen beträgt 83% mit einem *n*/*i*-Verhältnis von 40:60. Außerdem werden 10% an Ethylbenzol gefunden. 3-Phenylpropan-1-ol and 2-Phenylpropan-1-ol wurden nach der Extraktion des Lösungsmittels mit Wasser und anschließender Kugelrohr-Destillation (Siedepunkt 115-120 °C bei 1,8 kPa) in 80% Ausbeute als 2:3 Mischung erhalten.

*Beispiel 41 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Allylbenzol (2,6 mL; 2,4 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an entsprechenden Alkoholen beträgt 89% mit einem *n*/*i*-Verhältnis von 64:36. Außerdem werden 9% an *n*-Propylbenzol gefunden.

*Beispiel 42 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Isopren (2,0 mL; 1,4 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an entsprechenden Alkoholen beträgt >99% mit einem *n*/*i*-Verhältnis von 96:4. Die Zusammensetzung der Produkte ist: 3-Methyl-1-pentanol, 4-Methyl-1-pentanol und 2,3-Dimethyl-1-butanol (82:14:4).

*Beispiel 43 (Tabelle 4):*

Ein 25-mL Autoklav wird befüllt mit $Ru_3(CO)_{12}$ (25,6 mg; 40,0 μmol), 2-(Dicyclohexylphosphino)-1-methyl-1 H-imidazol (36,7 mg; 132 μmol) und LiCl (212 mg; 5,00 mmol). NMP (3 mL), $H_2O$ (1,0 mL; 1,0 g; 56 mmol) und Methacrylsäuremethylester (2,1 mL; 2,0 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 3-Methyldihydrofuran-2(3H)-on beträgt 74%.

**Komplexsynthese**

Tricarbon[$\eta^2$-2-(dicyclohexylphosphino-1-methyl-1*H*-imidazole]ruthenium(0) oder Tetracarbonyl[2-(dicyclohexylphosphino)-1-methyl-1*H*-imidazole]ruthenium(0) (K1)

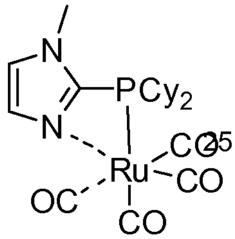

$Ru_3(CO)_{12}$ (102.4 mg, 160 μmol) und 2-(dicyclohexylphosphino)-1-methyl-1H-imidazole (127 mg, 456 μmol) wurden in Toluol (4 mL) gelöst und in einen Autoklaven überführt und es wurde Synthesegasdruck aufgepresst ($CO/H_2$ 1:1, 60 bar) und für 2 Stunden bei 100 °C gerührt. Nach dem Abkühlen wurde der Autoklave entspannt und die Reaktionsmischung wurde in einen Schlenkkolben unter Schutzgas überführt. Die Lösung wurde direkt mittels NMR untersucht.

$C_{20}H_{27}N_2O_4PRu$ (491.48 g/mol: 4CO) or $C_{19}H_{27}N_2O_3PRu$ (463.47 g/mol: 3CO)

**$^1$H-NMR** ($C_7D_8$, 300 MHz) δ/ppm: 1.00-1.27 (m, 6H, CyH), 1.30-1.45 (m, 4H, CyH), 1.48-1.56 (m, 2H, CyH), 1.58-1.71 (m, 4H, CyH), 1.77-1.85 (m, 2H, CyH), 1.88-1.96 (m, 2H, CyH), 2.51-2.62 (m, 2H, CyH), 3.50 (s, 3H, C$H_3$), 6.31 (d, $^2J_{HH}$ = 1.5 Hz, 1 H, Imid$H_5$), 7.05 (d, $^2J_{HH}$ = 1.5 Hz, 1H, Imid$H_4$).

**$^{13}$C{$^1$H}-NMR** ($C_7D_8$, 101 MHz) δ/ppm: 26.3 (*Cy*H), 27.0 (d, $J_{CP}$ = 11.7 Hz, *Cy*H), 27.2 (d, $J_{CP}$ = 13.5 Hz, *Cy*H), 28.1 (d, $J_{CP}$ = 1.8 Hz, CyH), 28.7 (*Cy*H), 35.5 (d, $^3J_{Cp}$ = 1.7 Hz, $CH_3$), 39.1 (d, $^1J_{CP}$ = 25.7 Hz, CyH), 125.8 (*Imid*$H_5$), 129.3 (d, $^3J_{CP}$ = 9.2 Hz, *Imid*$H_4$), 139.2 (d, $^1J_{CP}$ = 60.5 Hz, *Imid*P), 207.2 (d, $^2J_{CP}$ = 1.8 Hz, CO).

**$^{31}$P-NMR** ($C_7D_8$, 162 MHz) δ/ppm: 32.0.

<u>Tricarbonylbis[2-(dicyclohexylphosphino)-1-methyl-1*H*-imidazole]ruthenium(0) (K2)</u>

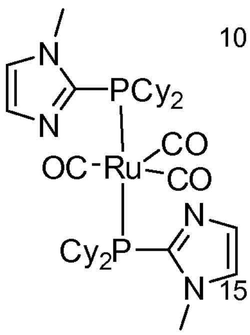

$Ru_3(CO)_{12}$ (102.4 mg, 160 μmol) und 2-(dicyclohexylphosphino)-1-methyl-1H-imidazole (146.8 mg, 864 μmol) wurden in Toluol (4 mL) gelöst und für 2 Stunden bei 100 °C gerührt. Die orangefarbene Lösung wurde auf Raumtemperatur abgekühlt und das Lösungsmittel wurde unter vermindertem Druck entfernt. Heptan (2 mL) wurde zugesetzt und der Rückstand wurde filtriert und mit Heptan. Als Produkt konnte ein hellgelber Feststoff erhalten werden (255 mg, 80%).

$C_{35}H_{54}N_4O_3P_2Ru$ (741.85 g/mol)

**$^1$H-NMR** ($C_7D_8$, 300 MHz) δ/ppm: 1.16-1.47 (m, 6H, CyH), 1.54-1.65 (m, 2H, CyH), 1.71-2.07 (m, 10H, CyH), 2.16-2.27 (m, 2H, CyH), 2.79-2.94 (m, 2H, CyH), 3.89 (s, 3H, C$H_3$), 6.38 (d, $^2J_{HH}$ = 1.0 Hz, 1H, Imid$H_5$), 7.12 (d, $^2J_{HH}$ = 1.0 Hz, 1H, Imid$H_4$).

**$^{13}$C{$^1$H}-NMR** ($C_7D_8$, 101 MHz) δ/ppm: 26.2 (*Cy*H), 27.6 (AA'X, $J_{CP}$ = 6.2 Hz, *Cy*H), 28.3 (*Cy*H), 29.3 (*Cy*H), 35.6 ($CH_3$), 41.6 (AA'X, $^1J_{CP}$ = 14.0 Hz, CyH), 125.5 (*Imid*$H_5$), 128.7 (*Imid*$H_4$), 141.8 (AA'X, $^1J_{CP}$ = 31.9 Hz, *Imid*P), 210.3 (t, $^2J_{CP}$ = 15.4 Hz, CO).

**$^{31}$P-NMR** ($C_7D_8$, 162 MHz) δ/ppm: 44.6.

<u>Katalyseexperimente mit Komplexen K1 und K2</u>

Tabelle 5: Ru-katalysierte Hydroformylierung/Reduktion von 1-Okten

| Bsp.[a] | Komplex | Ausbeute [%][b] | | |
|---|---|---|---|---|
| | | Alkohole (*n*/*i*) | Aldehyde (*n*/*i*) | Alkan |
| 44 | **K1** | 86 (89:11) | 3 | 8 |
| 45 | **K2** | 68 (88:12) | 1 | 4 |

[a] Soweit nicht anders beschrieben: 20,0 mmol 1-Okten, Komplex, 5,00 mmol LiCl, 1 mL $H_2O$, 3 mL NMP, 6,0 MPa $CO/H_2$ (1:1), 130 °C, 20 h in einem 25 mL Autoklav.
[b] Bestimmt mit GC mit einem internen Standard (2,0 mL Isooktan).

*Beispiel 44 (Tabelle 5):*

Ein 25-mL Autoklav wird befüllt mit Stammlösung des Komplexes in Toluol **K1** (c = 0,114 M; 1,0 mL; 120 μmol), und LiCl (212 mg; 5,00 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 86% mit einem *n*/*i*-Verhältnis von 89:11. Ausserdem werden 3% an C9-Aldehyden und 8% an Oktan gefunden.

*Beispiel 45 (Tabelle 5):*

Ein 25-mL Autoklav wird befüllt mit Komplex **K2** (89,4 mg; 120 μmol) und LiCl (212 mg; 5,00 mmol). NMP (3 mL), $H_2O$ (1,0 mL; 1,0 g; 56 mmol) und 1-Okten (3,1 mL; 2,2 g; 20 mmol) werden hinzugegeben und es wird Synthesegas ($CO/H_2$ 1:1 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an C9-Alkoholen beträgt 68% mit einem *n*/*i*-Verhältnis von 88:12. Außerdem werden 1% an C9-Aldehyden und 4% an Oktan gefunden.

Die Katalyseexperimente mit den Komplexen **K1** und **K2** zeigen nochmals, dass es sich bei der oben beschriebenen Carbonylierung von Olefinen um eine Reaktion handelt, welche mittels der entsprechenden Ruthenium-Ligand-Komplexe katalysiert wird.

Aus den Beispielen sind die sehr guten Ausbeuten und Selektivitäten für das erfindungsgemäße Verfahren deutlich erkennbar.

## Patentansprüche

1. Verfahren zur Carbonylierung von Olefinen, **dadurch gekennzeichnet, dass** wenigstens ein Olefin unter Zuführung von $H_2$ und CO in Gegenwart eines katalytischen Systems aus einem Ruthenium-Komplex und mindestens einem Liganden umgesetzt wird, wobei der Ligand einen organischen Phosphor-Liganden einer der beiden allgemeinen Formeln (I) oder (II) darstellt:

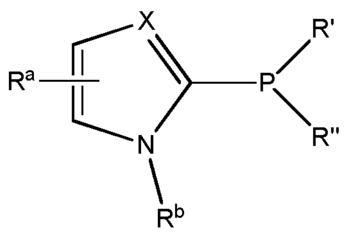

(I)

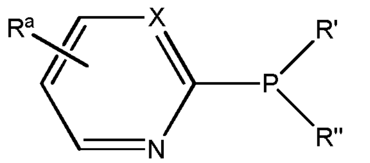

(II)

worin

R' und R" gleich oder verschieden sind und für einen Rest stehen, der ausgewählt ist aus der Gruppe bestehend aus:

$(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, O-$(C_1-C_{12})$-Alkyl, O-$(C_1-C_{12})$-Heteroalkyl, O-$(C_4-C_{14})$-Aryl, O-$(C_4-C_{14})$-Aryl-$(C_1-C_{14})$-Alkyl, O-$(C_3-C_{14})$-Heteroaryl, O-$(C_3-C_{14})$-Heteroaryl-$(C_1-C_{14})$-Alkyl, O-$(C_3-C_{12})$-Cycloalkyl, O-$(C_3-C_{12})$-Cycloalkyl-$(C_1-C_{12})$-Alkyl, O-$(C_3-C_{12})$-Heterocycloalkyl, O-$(C_3-C_{12})$-Heterocycloalkyl-$(C_1-C_{12})$-Alkyl, wobei die genannten Alkyl-, Heteroalkyl-, Cycloalkyl-, Heterocycloalkyl-, Aryl- und Heteroarylgruppen optional ein- oder mehrfach substituiert sind und

$R^a$ und $R^b$ ausgewählt sind aus: Wasserstoff, Hydroxy, $(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, O-$(C_1-C_{12})$-Alkyl, O-$(C_1-C_{12})$-Heteroalkyl, O-$(C_4-C_{14})$-Aryl, O-$(C_4-C_{14})$-Aryl-$(C_1-C_{14})$-Alkyl, O-$(C_3-C_{14})$-Heteroaryl, O-$(C_3-C_{14})$-Heteroaryl-$(C_1-C_{14})$-Alkyl, O-$(C_3-C_{12})$-Cycloalkyl, O-$(C_3-C_{12})$-Cycloalkyl-$(C_1-C_{12})$-Alkyl, O-$(C_3-C_{12})$-Heterocycloalkyl, O-$(C_3-C_{12})$-Heterocycloalkyl-$(C_1-C_{12})$-Alkyl,

wobei die genannten Alkyl-, Heteroalkyl-, Cycloalkyl-, Heterocycloalkyl-, Aryl- und Heteroarylgruppen optional ein- oder mehrfach substituiert sind und
und wobei $R^a$ auch zur Bildung eines größeren kondensierten Ringes befähigt ist, und X ausgewählt ist aus: N, C.

**2.** Verfahren nach Anspruch 1,
wobei der Ligand die allgemeine Formel (I) aufweist.

**3.** Verfahren nach Anspruch 1,
wobei der Ligand die allgemeine Formel (II) aufweist.

**4.** Verfahren nach einem der Ansprüche 1 bis 3,
wobei R' und R" ausgewählt sind aus: optional substituierten $(C_4-C_{14})$-Aryl-Rest, optional substituierten unverzweigten oder verzweigten aliphatischen Kohlenwasserstoffrest, optional substituierten Cycloalkylrest, optional substituierten Heteroarylrest.

**5.** Verfahren nach einem der Ansprüche 1 bis 3,
wobei R' und R" ausgewählt sind aus: $(C_1-C_8)$-Alkyl, $(C_4-C_{14})$-Aryl-O-$(C_1-C_8)$-Alkyl, $(C_4-C_{14})$-Aryl, Cyclohexyl, $(C_3-C_{14})$-Heteroaryl, $(C_3-C_{14})$-Heteroaryl-$(C_1-C_{12})$-Alkyl.

**6.** Verfahren nach einem der Ansprüche 1 bis 5,
wobei $R^a$ und $R^b$ ausgewählt sind aus: Wasserstoff, $(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, wobei die genannten Alkyl-, Heteroalkyl-, Cycloalkyl-, Heterocycloalkyl-, Aryl- und Heteroarylgruppen optional ein oder mehrfach substituiert sind,
und wobei $R^a$ auch zur Bildung eines größeren kondensierten Ringes befähigt ist.

**7.** Verfahren nach einem der Ansprüche 1 bis 5,
wobei $R^a$ und $R^b$ ausgewählt sind aus: Wasserstoff, $(C_1-C_{12})$-Alkyl, $(C_1-C_{12})$-Heteroalkyl, $(C_4-C_{14})$-Aryl,

$(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, wobei die genannten Alkyl-, Heteroalkyl-, Cycloalkyl-, Heterocycloalkyl-, Aryl- und Heteroarylgruppen optional ein- oder mehrfach substituiert sind
und wobei $R^a$ auch zur Bildung eines größeren kondensierten Ringes befähigt ist.

**8.** Verfahren nach einem der Ansprüche 1 bis 7,
wobei R' ausgewählt ist aus: Cyclohexyl, Phenyl, tert-Butyl und

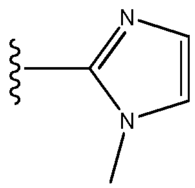

**9.** Verfahren nach einem der Ansprüche 1 bis 8,
wobei R" ausgewählt ist aus: Cyclohexyl, Phenyl, tert-Butyl und

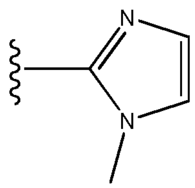

**10.** Verfahren nach einem der Ansprüche 1 bis 9,
**dadurch gekennzeichnet, dass** der Ligand ausgewählt ist aus der Gruppe bestehend aus:

2-(Dicyclohexylphosphino)pyridin (L1a),
2-(Diphenylphosphino)pyridin (L1b),
2-(Dicyclohexylphosphino)-1-methyl-1H-imidazol (L2a),
2-(Di-ter.butyl-phosphino)-1-methyl-1H-imidazol (L2b),
2-(Dicyclohexylphosphino)-1-(2-methoxyphenyl)-1H-imidazol (L2c),
2-(Dicyclohexylphosphino)-1-mesityl-1H-imidazol (L2d),
2-(Dicyclohexylphosphino)-1-methyl-1H-benzo[d]imidazol (L3).
2,2'-(Cyclohexylphosphinediyl)bis(1-methyl-1*H*-imidazol) (L4).

**11.** Verfahren nach einem der Ansprüche 1 bis 10,
**dadurch gekennzeichnet, dass** der Ruthenium-Katalysator in situ ausgehend von einem Vorkomplex gebildet wird, wobei als Rutheniumquelle Ruthenium-enthaltende Salze und Komplexe als Vorstufe verwendet werden, die Rutheniumcarbonylhydrid-Komplexe bilden.

**12.** Verfahren nach einem der Ansprüche 1 bis 11,
**dadurch gekennzeichnet, dass** es zur Umsetzung von Olefinen verwendet wird ausgewählt aus: Alkene, Cycloalkene, Carbonsäureester, aromatische Olefine mit jeweils einer Kohlenstoffanzahl von 2 bis 21.

**13.** Verfahren nach einem der Ansprüche 1 bis 12,
**dadurch gekennzeichnet, dass** bei einem Reaktionsdruck von 0,1 MPa bis 10,0 MPa gearbeitet wird.

**14.** Verfahren nach einem der Ansprüche 1 bis 13,
**dadurch gekennzeichnet, dass** das Verhältnis Ruthenium : Ligand im Bereich von 1 : 1 bis 1 : 50 liegt.

**15.** Verfahren nach einem der Ansprüche 1 bis 14,
**dadurch gekennzeichnet, dass** von 0,01 mol-% bis 0,5 mol-% Ruthenium bezogen auf das Olefin eingesetzt werden.

## Claims

1. Process for carbonylation of olefins, **characterized in that** at least one olefin is converted by feeding $H_2$ and CO in the presence of a catalytic system comprising a ruthenium complex and one or more than one ligand, wherein the ligand is an organophosphorus ligand of one of the two general formulae (I) and (II):

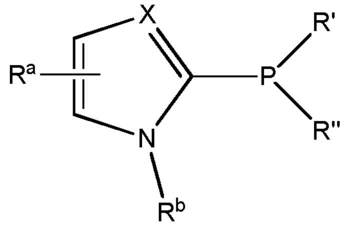

(I)

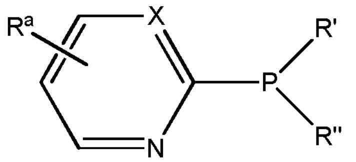

(II)

where

R' and R" are the same or different and are each a radical selected from the group consisting of:

($C_1$-$C_{12}$)-alkyl, ($C_1$-$C_{12}$)-heteroalkyl, ($C_4$-$C_{14}$)-aryl, ($C_4$-$C_{14}$)-aryl-($C_1$-$C_{12}$)-alkyl, ($C_4$-$C_{14}$)-aryl-O-($C_1$-$C_{12}$)-alkyl, ($C_3$-$C_{14}$)-heteroaryl, ($C_3$-$C_{14}$)-heteroaryl-($C_1$-$C_{12}$)-alkyl, ($C_3$-$C_{12}$)-cycloalkyl, ($C_3$-$C_{12}$) - cycloalkyl-($C_1$-$C_{12}$)-alkyl, ($C_3$-$C_{12}$)-heterocycloalkyl, ($C_3$-$C_{12}$)-heterocycloalkyl-($C_1$-$C_{12}$)-alkyl, O-($C_1$-$C_{12}$)-alkyl, O-($C_1$-$C_{12}$)-heteroalkyl, O-($C_4$-$C_{14}$)-aryl, O-($C_4$-$C_{14}$)-aryl-($C_1$-$C_{14}$)-alkyl, O-($C_3$-$C_{14}$) -heteroaryl, O-($C_3$-$C_{14}$)-heteroaryl-($C_1$-$C_{14}$)-alkyl, O-($C_3$-$C_{12}$)-cycloalkyl, O-($C_3$-$C_{12}$)-cycloalkyl-($C_1$-$C_{12}$)-alkyl, O-($C_3$-$C_{12}$)-heterocycloalkyl, O-($C_3$-$C_{12}$)-heterocycloalkyl-($C_1$-$C_{12}$)-alkyl,
wherein the recited alkyl, heteroalkyl, cycloalkyl, heterocycloalkyl, aryl and heteroaryl groups are optionally substituted one or more times, and

$R^a$ and $R^b$ are each selected from: hydrogen, hydroxyl, ($C_1$-$C_{12}$)-alkyl, ($C_1$-$C_{12}$) -heteroalkyl, ($C_4$-$C_{14}$)-aryl, ($C_4$-$C_{14}$)-aryl-($C_1$-$C_{12}$)-alkyl, ($C_4$-$C_{14}$)-aryl-O-($C_1$-$C_{12}$)-alkyl, ($C_3$-$C_{14}$)-heteroaryl, ($C_3$-$C_{14}$)-heteroaryl-($C_1$-$C_{12}$)-alkyl, ($C_3$-$C_{12}$)-cycloalkyl, ($C_3$-$C_{12}$)-cycloalkyl-($C_1$-$C_{12}$)-alkyl, ($C_3$-$C_{12}$)-heterocycloalkyl, ($C_3$-$C_{12}$)-heterocycloalkyl-($C_1$-$C_{12}$)-alkyl, O-($C_1$-$C_{12}$)-alkyl, O-($C_1$-$C_{12}$)-heteroalkyl, O-($C_4$-$C_{14}$)-aryl, O-($C_4$-$C_{14}$)-aryl-($C_1$-$C_{14}$)-alkyl, O-($C_3$-$C_{14}$)-heteroaryl, O-($C_3$-$C_{14}$)-heteroaryl-($C_1$-$C_{14}$)-alkyl, O-($C_3$-$C_{12}$)-cycloalkyl, 0-($C_3$-$C_{12}$)-cycloalkyl-($C_1$-$C_{12}$)-alkyl, O-($C_3$-$C_{12}$)-heterocycloalkyl, O-($C_3$-$C_{12}$)-heterocycloalkyl-($C_1$-$C_{12}$)-alkyl,

wherein the recited alkyl, heteroalkyl, cycloalkyl, heterocycloalkyl, aryl and heteroaryl groups are optionally substituted one or more times, and
wherein $R^a$ is also capable of forming a larger fused ring,
and X is selected from: N, C.

2. Process according to Claim 1,
wherein the ligand is of general formula (I).

3. Process according to Claim 1,
wherein the ligand is of general formula (II).

4. Process according to any of Claims 1 to 3,
wherein R' and R" are each selected from: optionally substituted ($C_4$-$C_{14}$)-aryl, optionally substituted branched or unbranched aliphatic hydrocarbyl, optionally substituted cycloalkyl, optionally substituted heteroaryl.

5. Process according to any of Claims 1 to 3, wherein R' and R" are each selected from: $(C_1-C_8)$-alkyl, $(C_4-C_{14})$-aryl-O-$(C_1-C_8)$-alkyl, $(C_4-C_{14})$-aryl, cyclohexyl, $(C_3-C_{14})$-heteroaryl, $(C_3-C_{14})$-heteroaryl-$(C_1-C_{12})$-alkyl.

6. Process according to any of Claims 1 to 5, wherein $R_a$ and $R^b$ are each selected from: hydrogen, $(C_1-C_{12})$-alkyl, $(C_1-C_{12})$-heteroalkyl, $(C_4-C_{14})$-aryl, $(C_4-C_{14})$-aryl-$(C_1-C_{12})$-alkyl, $(C_4-C_{14})$-aryl-O-$(C_1-C_{12})$-alkyl, $(C_3-C_{14})$-heteroaryl, $(C_3-C_{14})$-heteroaryl-$(C_1-C_{12})$-alkyl, $(C_3-C_{12})$-cycloalkyl, $(C_3-C_{12})$-cycloalkyl-$(C_1-C_{12})$-alkyl, $(C_3-C_{12})$-heterocycloalkyl, $(C_3-C_{12})$-heterocycloalkyl-$(C_1-C_{12})$-alkyl, wherein the recited alkyl, heteroalkyl, cycloalkyl, heterocycloalkyl, aryl and heteroaryl groups are optionally substituted one or more times, and wherein $R^a$ is also capable of forming a larger fused ring.

7. Process according to any of Claims 1 to 5, wherein $R^a$ and $R^b$ are each selected from: hydrogen, $(C_1-C_{12})$-alkyl, $(C_1-C_{12})$-heteroalkyl, $(C_4-C_{14})$-aryl, $(C_4-C_{14})$-aryl-$(C_1-C_{12})$-alkyl, $(C_4-C_{14})$-aryl-O-$(C_1-C_{12})$-alkyl, $(C_3-C_{14})$-heteroaryl, $(C_3-C_{14})$-heteroaryl-$(C_1-C_{12})$-alkyl, wherein the recited alkyl, heteroalkyl, cycloalkyl, heterocycloalkyl, aryl and heteroaryl groups are optionally substituted one or more times,
and wherein $R^a$ is also capable of forming a larger fused ring.

8. Process according to any of Claims 1 to 7, wherein R' is selected from: cyclohexyl, phenyl, tert-butyl and

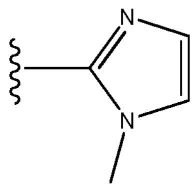

9. Process according to any of Claims 1 to 8, wherein R" is selected from: cyclohexyl, phenyl, tert-butyl and

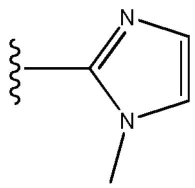

10. Process according to any of Claims 1 to 9, **characterized in that** the ligand is selected from the group consisting of:

2-(dicyclohexylphosphino)pyridine (L1a),
2-(diphenylphosphino)pyridine (L1b),
2-(dicyclohexylphosphino)-1-methyl-1H-imidazole (L2a),
2-(di-tert-butylphosphino)-1-methyl-1H-imidazole (L2b),
2-(dicyclohexylphosphino)-1-(2-methoxyphenyl)-1H-imidazole (L2c),
2-(dicyclohexylphosphino)-1-mesityl-1H-imidazole (L2d),
2-(dicyclohexylphosphino)-1-methyl-1H-benzo[d]imidazole (L3).
2,2'-(cyclohexylphosphinediyl)bis(1-methyl-1*H*-imidazole) (L4).

11. Process according to any of Claims 1 to 10, **characterized in that** the ruthenium catalyst is formed in situ from a pre-complex, wherein the ruthenium source used comprises ruthenium-containing salts and complexes as a precursor which form ruthenium carbonyl hydride complexes.

12. Process according to any of Claims 1 to 11, **characterized in that** it is used for conversion of olefins selected from: alkenes, cycloalkenes, carboxylic esters, aromatic olefins having in each case a carbon number of 2 to 21.

13. Process according to any of Claims 1 to 12, **characterized in that** it employs a reaction pressure of 0.1 MPa to 10.0 MPa.

**14.** Process according to any of Claims 1 to 13, **characterized in that** the ruthenium:ligand ratio is in the range from 1:1 to 1:50.

**15.** Process according to any of Claims 1 to 14, **characterized in that** from 0.01 mol% to 0.5 mol% of ruthenium based on the olefin is used.

**Revendications**

**1.** Procédé pour la carbonylation d'oléfines, **caractérisé en ce qu'**on transforme au moins une oléfine, avec addition de $H_2$ et de CO, en présence d'un système catalytique constitué par un complexe du ruthénium et au moins un ligand, le ligand étant un ligand phosphoré organique d'une des deux formules générales (I) ou (II):

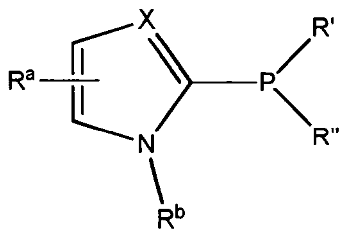

(I)

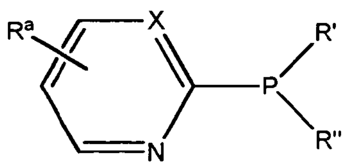

(II)

dans lesquelles

R' et R" sont identiques ou différents et représentent un radical qui est choisi dans le groupe constitué par : $(C_1-C_{12})$-alkyle, $(C_1-C_{12})$ -hétéroalkyle, $(C_4-C_{14})$-aryle, $(C_4-C_{14})$-aryl-$(C_1-C_{12})$ -alkyle, $(C_4-C_{14})$-aryl-O-$(C_1-C_{12})$ -alkyle, $(C_3-C_{14})$ -hétéroaryle, $(C_3-C_{14})$ -hétéroaryl- $(C_1-C_{12})$ -alkyle, $(C_3-C_{12})$-cycloalkyle, $(C_3-C_{12})$-cycloalkyl-$(C_1-C_{12})$ -alkyle, $(C_3-C_{12})$-hétérocycloalkyle, $(C_3-C_{12})$-hétérocycloalkyl-$(C_1-C_{12})$-alkyle, O-$(C_1-C_{12})$-alkyle, 0-$(C_1-C_{12})$ -hétéroalkyle, O-$(C_4-C_{14})$ -aryle, O-$(C_4-C_{14})$-aryl- $(C_1-C_{14})$ -alkyle, O-$(C_3-C_{l4})$ -hétéroaryle, O-$(C_3-C_{14})$-hétéroaryl-$(C_1-C_{14})$-alkyle, O-$(C_3-C_{12})$-cycloalkyle, O-$(C_3-C_{12})$-cycloalkyl-$(C_1-C_{12})$-alkyle, O-$(C_3-C_{12})$ -hétérocycloalkyle, O-$(C_3-C_{12})$-hétérocycloalkyl-$(C_1-C_{12})$-alkyle,
les groupes alkyle, hétéroalkyle, cycloalkyle, hétérocycloalkyle, aryle et hétéroaryle mentionnés étant éventuellement monosubstitués ou polysubstitués et
$R^a$ et $R^b$ sont choisis parmi : hydrogène, hydroxy, $(C_1-C_{12})$-alkyle, $(C_1-C_{12})$-hétéroalkyle, $(C_4-C_{14})$-aryle, $(C_4-C_{14})$-aryl-$(C_1-C_{12})$-alkyle, $(C_4-C_{14})$-aryl-O-$(C_1-C_{12})$-alkyle, $(C_3-C_{14})$-hétéroaryle, $(C_3-C_{14})$-hétéroaryl-$(C_1-C_{12})$-alkyle, $(C_3-C_{12})$-cycloalkyle, $(C_3-C_{12})$-cycloalkyl-$(C_1-C_{12})$-alkyle, $(C_3-C_{12})$-hétérocycloalkyle, $(C_3-C_{12})$-hétérocycloalkyl-$(C_1-C_{12})$-alkyle, O-$(C_1-C_{12})$-alkyle, O-$(C_1-C_{12})$-hétéroalkyle, O-$(C_4-C_{14})$ -aryle, O-$(C_4-C_{14})$-aryl- $(C_1-C_{14})$-alkyle, O-$(C_3-C_{14})$-hétéroaryle, O-$(C_3-C_{14})$-hétéroaryl-$(C_1-C_{14})$-alkyle, O-$(C_3-C_{12})$-cycloalkyle, O-$(C_3-C_{12})$-cycloalkyl-$(C_1-C_{12})$-alkyle, O-$(C_3-C_{12})$-hétérocycloalkyle, O-$(C_3-C_{12})$-hétérocycloalkyl-$(C_1-C_{12})$-alkyle,

les groupes alkyle, hétéroalkyle, cycloalkyle, hétérocycloalkyle, aryle et hétéroaryle mentionnés étant éventuellement monosubstitués ou polysubstitués et
et $R^a$ étant également apte à la formation d'un cycle condensé plus grand et X étant choisi parmi : N, C.

**2.** Procédé selon la revendication 1, le ligand présentant la formule générale (I).

**3.** Procédé selon la revendication 1, le ligand présentant la formule générale (II).

**4.** Procédé selon l'une quelconque des revendications 1 à 3, R' et R" étant choisis parmi : un radical $(C_4-C_{14})$-aryle éventuellement substitué, un radical hydrocarboné aliphatique, non ramifié ou ramifié, éventuellement substitué,

un radical cycloalkyle éventuellement substitué, un radical hétéroaryle éventuellement substitué.

**5.** Procédé selon l'une quelconque des revendications 1 à 3, R' et R" étant choisis parmi : ($C_1$-$C_8$)-alkyle, ($C_4$-$C_{14}$)-aryl-O-($C_1$-$C_8$) -alkyle, ($C_4$-$C_{14}$)-aryle, cyclohexyle, ($C_3$-$C_{14}$)-hétéroaryle, ($C_3$-$C_{14}$)-hétéroaryl-($C_1$-$C_{12}$)-alkyle.

**6.** Procédé selon l'une quelconque des revendications 1 à 5, $R^a$ et $R^b$ étant choisis parmi:

hydrogène, ($C_1$-$C_{12}$) -alkyle, ($C_1$-$C_{12}$) -hétéroalkyle, ($C_4$-$C_{14}$)-aryle, ($C_4$-$C_{14}$)-aryl-($C_1$-$C_{12}$)-alkyle, ($C_4$-$C_{14}$)-aryl-O-($C_1$-$C_{12}$)-alkyle, ($C_3$-$C_{14}$)-hétéroaryle, ($C_3$-$C_{14}$)-hétéroaryl-($C_1$-$C_{12}$) -alkyle, ($C_3$-$C_{12}$)-cycloalkyle, ($C_3$-$C_{12}$)-cycloalkyl-($C_1$-$C_{12}$)-alkyle, ($C_3$-$C_{12}$)-hétérocycloalkyle, ($C_3$-$C_{12}$)-hétérocycloalkyl-($C_1$-$C_{12}$)-alkyle, les groupes alkyle, hétéroalkyle, cycloalkyle, hétérocycloalkyle, aryle et hétéroaryle mentionnés étant éventuellement monosubstitués ou polysubstitués et $R^a$ étant également apte à la formation d'un cycle condensé plus grand.

**7.** Procédé selon l'une quelconque des revendications 1 à 5, $R^a$ et $R^b$ étant choisis parmi:

hydrogène, ($C_1$-$C_{12}$)-alkyle, ($C_1$-$C_{12}$)-hétéroalkyle, ($C_4$-$C_{14}$)-aryle, ($C_4$-$C_{14}$)-aryl-($C_1$-$C_{12}$)-alkyle, ($C_4$-$C_{14}$)-aryl-O-($C_1$-$C_{12}$)-alkyle, ($C_3$-$C_{14}$)-hétéroaryle, ($C_3$-$C_{14}$)-hétéroaryl-($C_1$-$C_{12}$)-alkyle, les groupes alkyle, hétéroalkyle, cycloalkyle, hétérocycloalkyle, aryle et hétéroaryle mentionnés étant éventuellement monosubstitués ou polysubstitués et $R^a$ étant également apte à la formation d'un cycle condensé plus grand

**8.** Procédé selon l'une quelconque des revendications 1 à 7, R' étant choisi parmi cyclohexyle, phényle, tert-butyle et

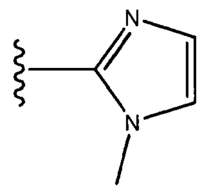

**9.** Procédé selon l'une quelconque des revendications 1 à 8, R" étant choisi parmi cyclohexyle, phényle, tert-butyle et

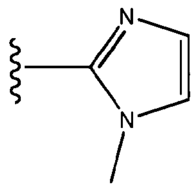

**10.** Procédé selon l'une quelconque des revendications 1 à 9, **caractérisé en ce que** le ligand est choisi dans le groupe constitué par: la 2-(dicyclohexylphosphino)pyridine (L1a), la 2-(diphénylphosphino)pyridine (L1b), le 2-(dicyclohexylphosphino)-1-méthyl-1H-imidazole (L2a), le 2-(di-tert-butylphosphino)-1-méthyl-1H-imidazole (L2b), le 2-(dicyclohexylphosphino)-1-(2-méthoxyphényl)-1H-imidazole (L2c), le 2-(dicyclohexylphosphino)-1-mésityl-1H-imidazole (L2d), le 2-(dicyclohexylphosphino)-1-méthyl-1H-benzo[d]imidazole (L3), le 2,2'-(cyclohexylphosphinediyl)bis(1-méthyl-1H-imidazole) (L4).

**11.** Procédé selon l'une quelconque des revendications 1 à 10, **caractérisé en ce que** le catalyseur du ruthénium est formé in situ partant d'un précomplexe, des sels et des complexes contenant du ruthénium étant utilisés comme source de ruthénium sous forme de précurseurs qui forment des complexes d'hydrure de ruthénium-carbonyle.

**12.** Procédé selon l'une quelconque des revendications 1 à 11, **caractérisé en ce qu'**il est utilisé pour la transformation d'oléfines choisies parmi: les alcènes, les cycloalcènes, les esters d'acide carboxylique, les oléfines aromatiques comprenant à chaque fois un nombre d'atomes de carbone de 2 à 21.

**13.** Procédé selon l'une quelconque des revendications 1 à 12, **caractérisé en ce qu'**on travaille à une pression de réaction de 0,1 MPa à 10,0 MPa.

**14.** Procédé selon l'une quelconque des revendications 1 à 13, **caractérisé en ce que** le rapport ruthénium:ligand se situe dans la plage de 1:1 à 1:50.

**15.** Procédé selon l'une quelconque des revendications 1 à 14, **caractérisé en ce qu'**on utilise 0,01% en mole à 0,5% en mole de ruthénium par rapport à l'oléfine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.,* 2012
- **P. KALCK et al.** *Adv. Organometal. Chem.,* 1991, vol. 32, 121-146
- *Angew. Chem. Int. Ed.,* 2012, vol. 51, 4383-4387